# EUROPEAN PATENT SPECIFICATION

(11) **EP 1 498 153 B1**
(45) Date of publication and mention of the grant of the patent: **26.08.2009**
(21) Application number: 04016655.5
(22) Date of filing: 15.07.2004
(51) Int. Cl.: A61N 7/02

(54) **Energy treatment apparatus**
Energiebehandlungsgerät
Appareil de traitement par énergie

(30) Priority: 15.07.2003 JP 2003197370; 28.07.2003 JP 2003202544; 31.07.2003 JP 2003205115
(43) Date of publication of application: 19.01.2005
(73) Proprietor: TERUMO KABUSHIKI KAISHA, Tokyo (JP)
(72) Inventor: Maki, Shin, Terumo Kabushiki Kaisha, Ashigarakami-gun Kanagawa 259-0151 (JP); Shiono, Hiroshi, Terumo Kabushiki Kaisha, Ashigarakami-gun Kanagawa 259-0151 (JP)
(74) Representative: Casalonga, Axel

(56) References cited:
- US-A- 5 344 435
- US-A- 5 664 570
- US-A- 6 007 499
- US-B1- 6 273 864

## Description

The present invention generally pertains to a medical treatment apparatus. More particularly, the invention relates to an energy treatment apparatus which is for example, adapted to be inserted into a blood vessel, a digestive tract such as esophagus, rectum, etc., or a cavity or lumen of a living body such as urethra, abdominal cavity, thoracic cavity, etc., or surgically pressed against a living body tissue or pressed against the body surface and which irradiates the body tissue with an energy such as ultrasound, a laser beam, etc. for the purpose of relatively high-temperature treatment of a tumor such as cancer, or BPH (benign prostatic hyperprasia) or the like.

Thermotherapeutic technologies (hyperthermia, high-temperature treatment, ablation, vaporization technique) are known in which an elongate insertion portion is inserted into a living body, either by utilizing a body cavity or by applying a small incision to the living body A living body tissue, including a lesion, is irradiated with energy such as ultrasonic waves or a laser beam from the insertion portion to annihilate the tissues at the lesion site or the surrounding tissues including the lesion site through alteration, sphacelation, coagulation, cauterization or vaporization, thereby achieving treatment.

In these technologies, generally, the treatment is carried out by directly irradiating the surface layer of the body tissue or the lesion site located in the vicinity thereof with the energy. The technologies are utilized also for thermotherapy of a lesion site located in the depth of a living body tissue such as, for example, the prostate.

U.S. Patent No. 6,379,347, U.S. Patent No. 6,562,029 and Japanese Application Publication No. 2000-319 disclose examples of a medical energy irradiation apparatus applied to such thermotherapy. U.S. Patent No. 5,743,863 and U.S. Patent No. 5,676,692 disclose treatment apparatus applied to high intensity focused ultrasound (HIFU) therapy.

When a medical energy irradiation apparatus is to be applied to the treatment of the prostate, for example, the treatment is generally conducted in the following procedure. The doctor treating the prostate inserts an insertion portion of the medical energy irradiation apparatus into the rectum, sets the position of an emission unit to the prostate, adjusts the position of the emission unit toward the target site (the site to be treated), and irradiates the target site with the energy. This series of operations is generally performed by the doctor while observing the treatment condition through ultrasonic image diagnosis.

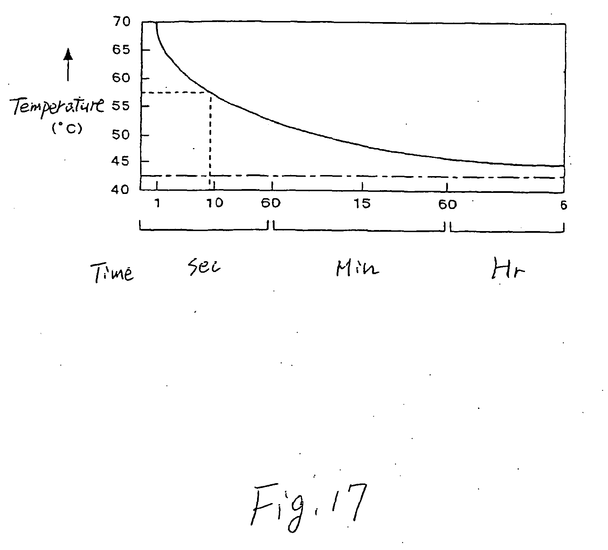

Fig. 17 is a graph showing the relationship between temperature and time for producing irreversible damage (sphacelation) to a living body tissue. The living tissue undergoes sphacelation in a shorter time as the temperature is higher. For example, to bring a tissue to sphacelation, it takes 30 seconds at a temperature of 55°, and one hour at a temperature of 45° (Lasers in Surgery and Medicine, 18: 22-23, 1996)).

The above-mentioned conventional energy irradiation apparatus for thermotherapy is configured to concentrate irradiation at the target site with the energy, for example, the laser beam emitted from the emission unit. In order to treat the target site while maintaining the target site in the above-mentioned heated state at the time of laser irradiation, it is necessary to enhance the utilization efficiency of energy. To enhance the energy utilization efficiency, it is necessary to raise the output of emission, which may produce damage to normal tissues (i.e., tissue other than at the target site).

US 5664570 discloses a device according to the preamble of appended claim 1.

A need thus exists for an energy treatment apparatus which is better suited to enhancing the utilization efficiency of energy emitted from an emission unit while also enhancing the accumulation of energy at the target site and suppressing the emission output.

As claimed, an energy treatment apparatus for irradiating living body tissue with energies comprises energy emission means for emitting energy toward a target site of living body tissue, wherein the energy emission means are adapted to emit energy having first and second different frequencies.

According to the invention, reflection means are positioned relative to the energy emission means toward said target site of living body tissue. The energy emission means and the reflection means are configured according to one of the following alternatives (i), (ii) or (iii):
(i) the energy emission means comprises a first respectively a second ultrasonic oscillator disposed side by side within one applicator, the reflection means is disposed at a position opposite to the applicator such that a first respectively a second standing wave is produced by superposition of first respectively second ultrasound radiated from the first respectively second ultrasonic oscillator with respective ultrasound reflected from the reflection means;
(ii) the energy emission means comprises a first annular respectively a second circular ultrasonic oscillator disposed concentric with one another within one applicator, the reflection means is disposed at a position opposite to the applicator such that a first respectively a second standing wave is produced by superposition of first respectively second ultrasound radiated from the first respectively second ultrasonic oscillator with respective ultrasound reflected from the reflection means;
(iii) the energy emission means and the reflection means are embodied in two assemblies each including an emission means and reflection means arranged in integral form, the two assemblies being arranged at positions opposite to one another, the first respectively second assembly each comprises an ultrasonic oscillator and a reflector located in surrounding relation to the ultrasonic oscillator, whereby a first standing wave is produced by the ultrasonic oscillator in the first assembly and the reflector in the second assembly and a second standing wave is produced by the ultrasonic oscillator in the second assembly and the reflector in the first assembly.

Further according to the invention, in each of the alternatives (i), (ii) and (iii) the first and second standing wave are superposed on each other to give a maximum intensity at the target site; and the first standing wave and the second standing wave having different frequencies from one another.

In the energy treatment apparatus according to the invention, the first and second wavelengths (λ1, λ2) corresponding to the first and second frequency (f1, f2) may each be defined as
wavelength = acoustic velocity/frequency,
and, in order to obtain a highest ultrasound intensity at the centre of the distance (L) between the emission unit and the reflector are set so as fulfil the condition L = 1 / (2×( | 1/λ1-1/λ2 |)).

In the energy treatment apparatus according to the invention, the distance between the emission unit and the reflector may be L, the wavelengths (λ1, λ2) of the respective radiated energies are so set that an integer times one half of the wavelength of each of the standing waves are equal to the distance (L) between the emission unit and the reflector, i.e. L=n×λ1/2 and L=m×λ2/2, where n and m are integers.

In the energy treatment apparatus according to the invention, where the wavelengths (λ1, λ2) of the respective radiated energies are present or known, the distance (L) between the emission unit and the reflector may be controlled so that the wavelengths (λ1, λ2) satisfy the formulas L=n×λ1/2 and L=m×λ2/2, where n and m are integers.

In the energy treatment apparatus according to the invention, the energy emission unit may comprise several emission units for emitting different-frequency energies.

Various illustrative examples of energy treatment apparatuses, which are not according to the present invention but which are for use in irradiating a living body tissue with energy, are also described herein.

According to one aspect, such illustrative example apparatus can includes an energy emission unit for emitting energy to irradiate a target site of living body tissue, and reflection means disposed at a position opposite to the energy emission unit to reflect at least some of the energy emitted from the energy emission unit toward the target site.

According to another aspect, such illustrative example energy treatment apparatus for irradiating living body tissue with energy comprises an energy emission unit for emitting energy toward a target site of living body tissue, reflection means disposed at a position opposite to the energy emission unit to reflect energy emitted from the energy emission unit toward the target site, and moving means for moving the energy emission unit to vary a position of the energy emission unit.

Another aspect involves an illustrative example energy treatment apparatus for irradiating living body tissue with energy comprising energy emission means for emitting energy toward a target site of living body tissue along a plurality of different routes, and reflection means disposed in opposing relation to the energy emission means for receiving at least some of the energy emitted along the different routes from the energy emission means and for reflecting at least some of the energy emitted along the different routes from the energy emission means towards the target site.

In accordance with another aspect, an illustrative example energy treatment apparatus for irradiating living body tissue with energies comprises a first energy emission unit for emitting energy having a first frequency toward a target site of living body tissue, a second energy emission unit for emitting energy having a second frequency different from the first frequency toward the target site of living body tissue, and reflection means positioned relative to the first and second energy emission units for reflecting at least a portion of the energy of the first and second energy emission units toward the target site of living body tissue so that emitted energy from the first energy emission unit and reflected energy from the first emission unit that is reflected by the reflecting means produces a first standing wave between the first emission unit and the reflection means, and emitted energy from the second energy emission unit and reflected energy from the second energy emission unit that is reflected by the reflecting means produces a second standing wave between the second emission unit and the reflection means.

An additional aspect involves an illustrative example energy treatment apparatus for irradiating living body tissue with energies comprising energy emission means for emitting energy having first and second different frequencies toward a target site of living body tissue, and reflection means positioned relative to the energy emission means to reflect at least a portion of emitted energy emitted by the energy emission means toward the target site of living body tissue. According to a further aspect, an energy treatment apparatus for irradiating living body tissue with energy comprises energy emission means for emitting energy to irradiate a target site of living body tissue, reflection means disposed relative to the energy emission means to reflect at least a portion of emitted energy emitted from the energy emission means toward the target site, and control means for controlling the energy emission means so that reflected energy reflected by the reflecting means and the emitted energy emitted by the energy emission means are superposed on one another at the target site.

In accordance with another aspect, an illustrative example energy treatment apparatus for irradiating living body tissue with energy comprises a transuretheral energy emission unit for emitting energy to irradiate a target site of a living body tissue and a transrectal reflection means disposed relative to the transuretheral energy emission unit to reflect at least a portion of emitted energy emitted from the transuretheral energy emission unit toward the target site, with the target site being sandwiched between the transuretheral energy emission unit and the transrectal reflection means.

By disposing reflection means at a position opposite to the emission unit, it is possible to utilize both the energy from the energy emission unit and the energy reflected by the reflection means. In addition, it is possible to irradiate the target site with the energy from the energy emission unit, and to irradiate the target site with both the energy partly penetrating through the target site and reflected by the reflection means and the energy from the energy emission unit. Therefore, it is possible to efficiently enhance the energy accumulation density at the target site, and to weaken the energy accumulation density at body tissues other than the target site. The emitted energy emitted from the energy emission unit and the reflected energy reflected from the reflection means can be superposed on each other at the target site.

An arranging apparatus can also be provided to arrange both a main body including the energy emission portion and the reflection means. The arranging apparatus preferably includes a holding unit for the main body including the energy emission unit, a holding unit for the reflection means, and a mechanism for regulating the relative positions of the energy emission unit and the reflection means. In addition, an arrangement control means can be provided for controlling the arranging apparatus. The arrangement control means preferably performs control such that the emitted energy emitted from the energy emission unit and the reflected energy reflected from the reflection means are superposed on each other at the target site.

The arranging apparatus can be configured to change the direction of the energy emission unit, and can be provided with reflection control means for controlling the reflection means. The energy treatment apparatus can also include an emission control means for controlling the emitted energy from the energy emission unit. The emission control means can control the irradiation pattern for concentrating the emitted energy into the target site, the irradiation output, and the irradiation time.

When provided, the emission control means preferably controls the irradiation pattern in such a way that the target site is intermittently irradiated with the emitted energy from the emission unit. Also, the emission control means can preferably control the irradiation pattern so that the energy intermittently emitted from the energy emission unit and the reflected energy reflected by the reflection means reach the target site simultaneously.

In a further illustrative example, the reflection means can include a fixed reflective surface or a variable reflective surface, and can be configured so that the curvature of the reflective surface and its radius of curvature are variable so as to efficiently reflect the energy. The reflection means can be provided with an energy detection means for detecting the quantity of energy reaching the reflection means from the energy emission unit. The reflection means can also be provided with air bleeding means for discharging air between the reflection means and living body tissue.

The energy treatment apparatus can additionally include cooling means for cooling at least one of the energy emission unit and living body tissue contacted by a main body including the energy emission unit. Cooling means can also be provided for cooling at least one of the reflection means and living body tissue contacted by the reflection means. Additionally, cooling control means can be employed to control the cooling means.

A controller can be provided for controlling the movement control means. Also, a controller can be provided to control the arrangement control means or to control both the arrangement control means and the emission control means. A controller can control the reflection control means or can control at least two of the reflection control means, the arrangement control means, and the emission control means. Also a controller can control the cooling control means or can control at least two of the cooling control means, the arrangement control means, the emission control means, and the reflection control means.

The energy treatment apparatus is preferably configured so that a main body including the energy emission unit includes means for converging, collimating, or diverging the spreading angle of the emitted energy. The main body having the energy emission unit can also be provided with observation means for positioning and for observing the inside of a living body.

Also, a balloon capable of expansion and contraction for position fixation can be provided at the surface of the main body including the energy emission unit or at the surface of the reflection means, or at the surface of both the main body and the reflection means.

A surface layer including a hydrophilic polymer material can be provided at the surface of a main body having the energy emission unit or at the surface of the reflection means, or at the surface of both the main body and the reflection means.

The form of energy which can be employed includes ultrasounds, laser beams and others. Ultrasound energy has depth-reaching capability and is thus beneficial to use in cases where the energy treatment apparatus is applied, for example, to the prostatic treatment or the like which requires or benefits from the depth-reaching capability of the energy.

The foregoing and additional features will become more apparent from the following detailed description considered with reference to the accompanying drawing figures in which like elements bear like reference numerals.
Fig. 1 is a side view somewhat schematically illustrating features associated with an energy treatment apparatus according to an illustrative example.
Fig. 2A is a side view of an essential part of the energy treatment apparatus of Fig. 1.
Fig. 2B is a cross-sectional view taken alone the section line 2B-2B in Fig. 2A.
Figs. 3A-3E are schematic illustrations of the energy (ultrasound) propagation conditions in an energy irradiation pattern in the energy treatment apparatus disclosed here.
Fig. 4A is a graph showing heating for illustrating one example of the energy irradiation pattern in the energy treatment apparatus disclosed here.
Fig. 4B is an illustration of one example of pulse trains associated with the energy treatment apparatus.
Fig. 5 is a cross-sectional view of another example of the energy irradiation pattern in the energy treatment apparatus described here.
Fig. 6 is a schematic illustration of another illustrative example of an energy treatment apparatus.
Fig. 7A is a schematic illustration of another illustrative example of an ultrasound conversion device applied to an illustrative example energy treatment apparatus.
Fig. 7B is a schematic illustration of another illustrative example of the ultrasound conversion device applied to the illustrative example energy treatment apparatus.
Fig. 8 is a perspective view of an illustrative example of a reflection means used in the illustrative example energy treatment apparatus.
Fig. 9A is a perspective view of another version of the illustrative example reflection means.
Fig. 9B is a cross-sectional view of the reflection means taken along the section line 9B-9B of Fig. 9A.
Fig. 10A is a perspective view of another embodiment of the reflection means.
Fig. 10B is a cross-sectional view of the reflection means taken along the section line 10B-10B of Fig. 10A.
Fig. 11A is a cross-sectional view of another embodiment of the reflection means.
Fig. 11B is a cross-sectional view of a still further embodiment of the reflection means.
Fig. 11C is a cross-sectional view of an additional embodiment of the reflection means.
Fig. 12A is a cross-sectional view of another embodiment of the reflection means.
Fig. 12B is a side view of the reflection means shown in Fig. 12A.
Figs. 13A and 13B illustrate the operations of the reflection means shown in Figs. 12A and 12B.
Fig. 14 is a perspective view of the overall configuration of one embodiment of the energy treatment apparatus disclosed here.
Fig. 15 is a flowchart illustrating one embodiment of a treatment conducted by use of the illustrative example energy treatment apparatus described here.
Fig. 16 is a flowchart illustrating one embodiment of position adjustment as to the reflector used in the illustrative example energy treatment apparatus.
Fig. 17 is a graph relating temperature and time in the case of a known energy treatment apparatus.
Fig. 18 is a schematic illustration of another illustrative example of an energy treatment apparatus.
Fig. 19 schematically illustrates a part of an energy emission portion of the energy treatment apparatus generally shown in Fig. 18.
Fig. 20A is a perspective view of a portion of the energy emission unit shown in Fig. 19.
Fig. 20B is a cross-sectional view of the energy emission unit of Fig. 20A.
Fig. 21A is an illustration of the operation of the energy treatment apparatus of Fig. 18.
Fig. 21B is an illustration of the operation of the energy treatment apparatus of Fig. 18 using another energy emission unit.
Fig. 22 is a perspective view of an overall configuration of another illustrative example of an energy treatment apparatus.
Fig. 23 is a graph showing the results of calculation of energy accumulation in the energy treatment apparatus of Fig.22.
Figs. 24A to 24C schematically show other illustrative examples of the energy emission unit.
Fig. 25 is an illustration of the operation of the energy emission unit shown in Figs. 24A-24C.
Fig. 26 is a schematic illustration of a moving means for the energy emission unit.
Fig. 27 is a schematic illustration of another illustrative example of an energy treatment apparatus.
Fig. 28 is a schematic illustration of a first embodiments of the energy treatment apparatus according to the present invention.
Fig. 29 is a schematic illustration of the propagation condition of the energies (e.g., ultrasounds) which are standing waves in the energy irradiation pattern in the energy treatment apparatus shown in Fig. 28.
Fig. 30 is a graph showing the relationship between distance and a second frequency in the energy treatment apparatus shown in Fig. 28.
Fig. 31A is an illustration of one example of the energy irradiation pattern associated with the energy treatment apparatus.
Fig. 31B is an illustration of the energy irradiation pattern of Fig. 31A.
Fig. 32A is an illustration of one example of the emission units which can be used in the energy treatment apparatus according to the invention generally shown in Fig. 28.
Fig. 32B is an illustration of another example of the emission units which can be used in the energy treatment apparatus according to the invention generally shown in Fig. 28.
Fig. 33 is a cross-sectional view illustrating still another example of the energy irradiation pattern associated with another embodiment of an energy treatment apparatus according to the invention.
Fig. 34 is an illustration of a further embodiment of the energy irradiation pattern associated with the energy treatment apparatus according to the invention.
Fig. 35 is a schematic illustration of still another embodiment of the energy treatment apparatus according to the present invention.

The energy treatment apparatus 1 according to an illustrative example comprises an applicator 2 in the form of a generally elongated insertion portion, an energy emission unit 3 disposed in the applicator to irradiate living body tissue with energy, a reflection means 4 disposed opposite the emission unit 3 to reflect the energy emitted from the emission unit 3, and a controller 5 which comprises a control unit for designating an irradiation pattern for concentrating the energy into or at a target site, or site to be treated, 80 of a living body tissue, and other control units. The reflection means 4 is comprised of a reflector 6 having a flat surface or concave surface for substantially reflecting the energy, and an operating rod 7 for inserting the reflector 6. In the illustrated version, the reflector 6 is integrally provided at the distal end of the operating rod 7.

The emitted energy can take a variety of forms, including ultrasounds, laser beams, electromagnetic waves having directivity, etc. The energy treatment apparatus 1 in the embodiments described here is applied as an ultrasonic treatment apparatus using ultrasounds having a relatively high depth-reaching capability as the energy. Therefore, an ultrasonic oscillator constituting the emission unit 3 is provided on the distal end side in the applicator 2. Ultrasound is emitted from the ultrasonic oscillator 3. As examples of the ultrasonic oscillator, devices can be employed whose shapes are varied upon application of voltages thereto, such as piezoelectric ceramics (PZT, barium titanate, etc.), piezoelectric polymers (PVDF, P(VDF-TriFE), etc.) and the like.

The irradiation pattern designated or set by the controller 5 can be such that, by way of cooperation of the ultrasound emitted from the ultrasonic oscillator 3 with the reflected ultrasound reflected by the reflector 6, the intensity of the ultrasound is higher at the target site 80 and is lower at other sites. In this embodiment, the ultrasound is radiated from the ultrasonic oscillator 3 toward the target site 80, and a part of the ultrasound penetrates through the target site 80 and is reflected by the reflector 6 so that the reflected ultrasound is again radiated to the target site 80.

As will be described below in more detail, the applicator 2 and the reflection means 4 are arranged to be movable so that the distance therebetween can be controlled. Therefore, they are each preferably formed of a material having a comparatively high hardness; for example, they may be formed of stainless steel or the like. Materials for constituting the applicator 2 will be described later.

An example of the principle associated with the use of the energy treatment apparatus 1, or ultrasonic treatment apparatus, according to this illustrative example will be described. The following description will consider an example in which the energy treatment apparatus 1 is applied to the treatment of prostatic cancer or BPH.

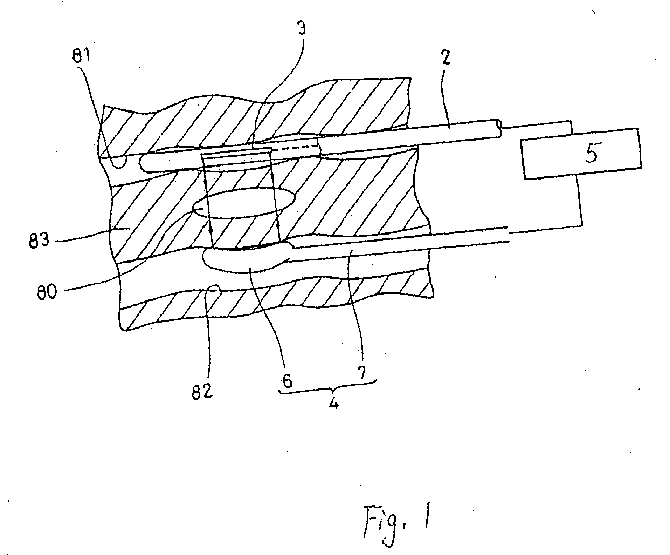

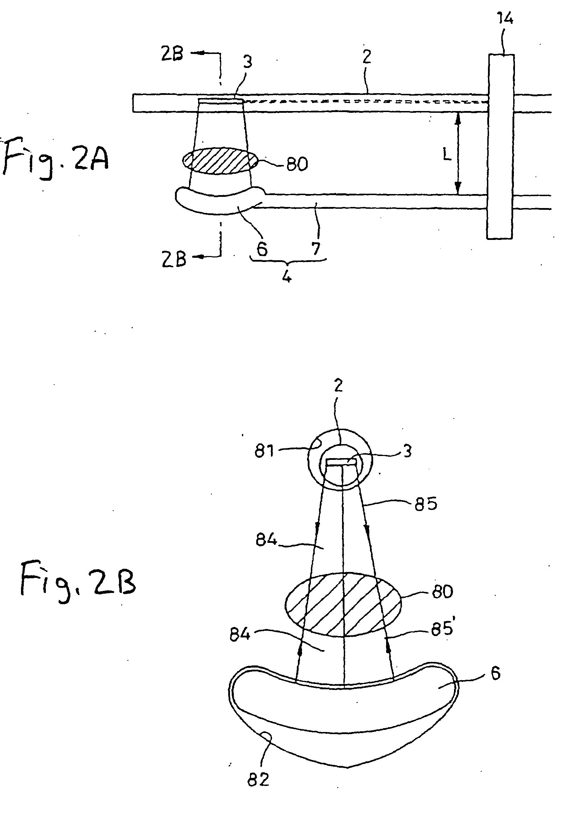

As shown in Fig. 1 and Figs. 2A and 2B, the applicator 2 is inserted into the urethra 81, whereas the reflector 6 is inserted into the rectum 82 through the use of the operating rod 7 so that the reflector 6 is transrectally located at a position corresponding to, or in opposing relation to, the applicator 2. The applicator 2 is transurethrally inserted or positioned so that the ultrasonic oscillator 3 provided on the distal end side is located at a position corresponding to or directed at the target site 80 of the prostate 83. Therefore, the prostate 83 is sandwiched between the applicator 2 and the reflector 6. In this case, for example, by observing the urethra through an endoscope provided in the applicator 2, it is possible to find out the shape characteristics of the urethra at the prostate area and to judge the forward-backward position of the target site 80. In this condition, the ultrasound 85 is intermittently emitted from the ultrasonic oscillator 3 toward the target site 80. The target site 80 is irradiated with the ultrasound 85 thus emitted. In addition, a part of the emitted ultrasound 85 penetrates through the target site 80, is directed to the reflector 6 inserted in the rectum 82 and is reflected by the reflector 6. This ultrasound 85' reflected from the reflector 6 is again utilized for irradiation of the target site 80. The target site 80 is thus irradiated with both the ultrasound 85 coming directly from the ultrasonic oscillator 3 and the ultrasound 85' reflected from the reflector 6; thus, the ultrasound 85 directly from the oscillator 3 and the reflected ultrasound85' are superposed on each other, and so the intensity of the ultrasonic energy directed at the target site 80 is augmented or strengthened. Since the intensity of the ultrasonic energy is weak at sites 84 other than the target site 80, the normal living tissues are not damaged and are generally maintained in the normal condition. Therefore, it is possible to selectively alter the tissue cells at the target site 80, for example, a malignant tumor of prostatic cancer or BPH, and to selectively treat the prostate.

In the present embodiment, the ultrasonic energy is accumulated at the target site 80 in such a manner that the temperature at the target site 80 is controlled to within a temperature range in which a high-temperature treatment can be performed and, yet, vaporization is not caused, preferably about 55°C to 100°C, more preferably about 70°C to 90°C.

As shown in Fig. 2A, the applicator 2 and the reflection means 4 are fixed to a holding unit 14 so that the distance L therebetween is set to a required distance.

The irradiation pattern designated by the controller 5, in the present embodiment, is such a pattern that the ultrasound 85 from the ultrasonic oscillator 3 is intermittently radiated to the target site 80, a part of the ultrasound penetrates through the target site 80 and is reflected by the reflector 6, with the reflected ultrasound 85' and the next ultrasound 85 from the ultrasonic oscillator 3 being synchronously radiated to the target site 80. Namely, the ultrasound 85 from the ultrasonic oscillator 3 and the reflected ultrasound 85' are superposed on each other at the target site 80.

One example of the irradiation pattern will now be described. In the present example, the ultrasound pulse train 85 is oscillated in a pulsed form from the ultrasonic oscillator 3, and the next ultrasound pulse train 85 is radiated to the target site 80 synchronously with the arrival at the target site 80 of the reflected ultrasound 85' reflected by the reflector 6 so that the two ultrasounds (i.e., the reflected ultrasound 85' from one pulse train and the direct ultrasound 85 from the next pulse train) are superposed on each other at the target site 80, resulting in enhanced intensity of the ultrasound.

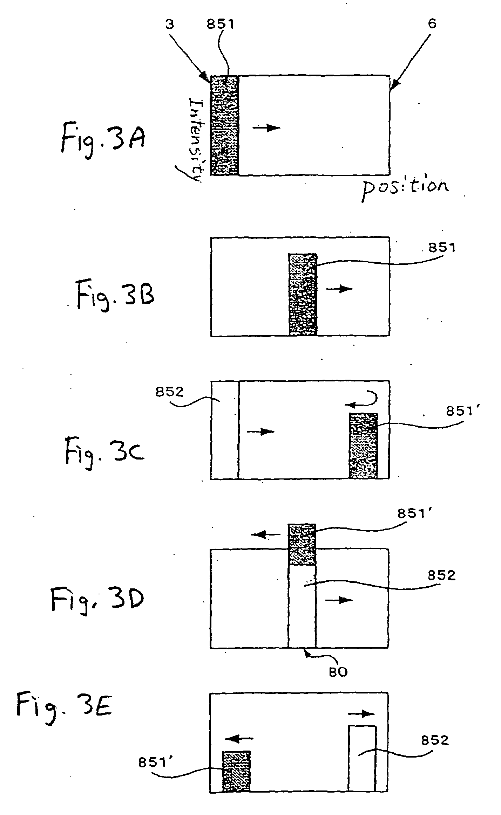

Fig. 3 schematically shows the ultrasound propagation condition of the irradiation pattern. In Fig. 3A, the first ultrasound pulse train 851 radiated from the side of the emission unit 3 is emitted. In Fig. 3B, the first ultrasound pulse train 851 is propagated to the reflector 6 through the change of the position thereof while giving energy. In Fig. 3C, the second ultrasound pulse train 852 is emitted, and the first ultrasound pulse train 851' reflected by the reflector 6 is propagated as shown. The timing of the emission of the second ultrasound pulse train 852 is set so that the second pulse train 852 will be superposed on the reflected pulse train 851' at the position where energy density is desired to be enhanced 80 (e.g., the target site). In Fig. 3D, the second ultrasound pulse train 852 and the reflected pulse train 851' arrive simultaneously at the position where energy density is to be enhanced, whereby the energy density is enhanced. In Fig. 3E, the second ultrasound pulse train 852 and the reflected first pulse train851' are propagated in the opposite directions. The intensity of the pulse train 851' having passed through the position 80 after reflection is attenuated as shown. From then on, the processes shown in Figs. 3C-3E are repeated, whereby a high energy density is achieved at the target site.

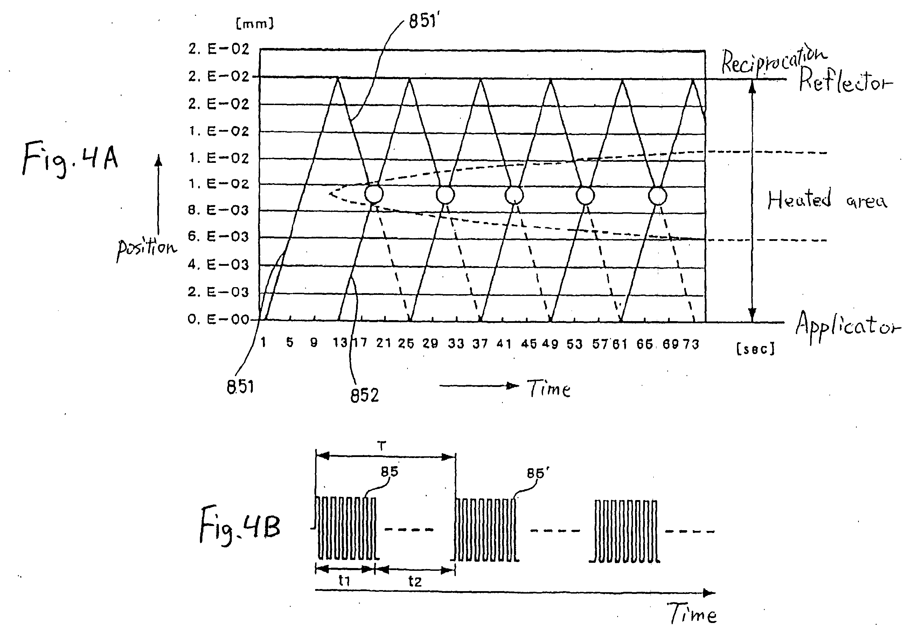

Fig. 4A shows the heated state of the target site heated by this irradiation pattern. Specifically, Fig. 4A is a graph showing the heated state of the target site heated by ultrasound, in which time is taken along the abscissa (x-axis), and the distance between the emission unit and the reflector is taken along the ordinate (y-axis).

The first ultrasound pulse train (fundamental wave) 851 radiated from the emission unit 3 is reflected by the reflector 6, and then reaches the target site 80. The second ultrasound pulse train (delayed wave) 852 is radiated at such a timing that 9 it will be superposed on the reflected first ultrasound pulse train 851' at the target site 80, namely, at the position of circles in the graph, resulting in enhancement of the energy density.

Fig. 4A shows the case where the target site 80 is located at a distance equal to 1/2 the distance between the applicator 2 and the reflector 6, wherein the next pulse train is emitted from the ultrasonic oscillator 3 with a delay of half phase, so that both ultrasound pulse trains are superposed on each other in the vicinity of the center. The timing of the emission of the pulse trains can be set as necessary according to the position of the target site. For example, the pulse separation can be larger as the target site 80 is nearer to the applicator 2, and the pulse separation can be smaller as the target site 80 is nearer to the reflector 6. In addition, the pulse width can be varied according to the width of the target site. The treatment region can be selected by setting the pulse separation (period) and the pulse width of the ultrasound pulse trains. The region indicated by the broken line in Fig. 4A is the heated region.

Next, referring to Fig. 4B, the ultrasound pulse trains intermittently emitted from the emission unit will be described. The pulse train 85 is emitted periodically. With the cyclic period represented as time T, the emission time of the pulse train 85 represented as time t1, the distance between the emission unit and the reflector represented as L, and the speed of sound represented as v, then T = 2L/v, and the intermittent energy emission time t1 is in the range of 0 < t1 < T/2. In the Figure, t2 denotes the rest time during which no pulse train is emitted.

Here, in the case of ultrasound, a high-temperature treatment can be performed under the following conditions.
1. The frequency of ultrasound is in the range of from 100 kHz to 50 MHz, more desirably from 1 to 5 MHz.
2. The distance L from the emission unit to the reflector is in the range of from 0.1 to 10 cm, more desirably from 1 to 6 cm.
3. The range of wavelength, in view of λ = v/f, v = 1530 m/sec and the frequency in condition 1, is from 15 to 0.03 mm, more desirably from 1.5 to 0.3 mm.
4. The cyclic period T, in view of condition 1 and condition 2, is in the range of from 1.3 to 130 µsec, more desirably from 13 to 80 µsec.
5. The pulse emission time t1 can be in the range of from 1/20 to 1/2 times the cyclic period T, more desirably from 1/10 to 1/3 times the cyclic period T.

Specifically, when the range of T is from 1.3 to 130 µsec, the range of t1 is from 1.3 µsec × 1/20 to 130 µsec × 1/2, more desirably from 1.3 µsec × 1/10 to 130 µsec × 1/3. When the range of T is from 13 to 80 µsec, the range of t1 can be from 13 µsec × 1/20 to 80 µsec × 1/2, more desirably from 13 µsec × 1/10 to 80 µsec × 1/3.

While ultrasound has been used in the above-mentioned example, the preferred or optimum conditions can be determined according to the purpose in the cases of performing a high-temperature treatment, for example by using lasers, electromagnetic waves, or the like.

Another example of the irradiation pattern will now be described. In this example, ultrasound is continuously oscillated from the ultrasonic oscillator 3, and the ultrasonic oscillator 3 is rotated about the axis of the applicator 2 so that the target site will be irradiated, apparently in a pulsed mode, with the ultrasound each time the ultrasonic oscillator 3 is rotated through one revolution. A high-temperature treatment using this irradiation pattern is preferable when applied, for example, to the treatment of the whole perimeter of the prostate in the case where sites other than the critical site of prostatic cancer are suspicious.

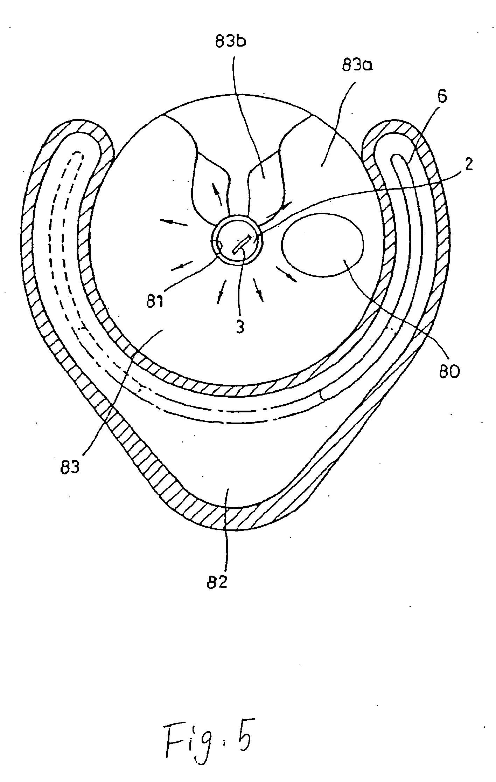

Fig. 5 shows a cross-section of the applicator 2 of the ultrasound treatment apparatus 1 and the prostate 83, in the case where this irradiation pattern is applied. The prostate 83 is composed of a peripheral region 83a and a transition region 83b. Here, the treatment is applied to the whole perimeter of the prostate including the critical site 80 of prostatic cancer. In this example, the ultrasonic oscillator 3 is disposed in the applicator 2 so that it can be rotated with a required period. On the other hand, the rectum 82 into which the reflector 6 is inserted is a very flexible organ, and can be extended to the whole region of the peripheral region 83a exclusive of the transition region 83b of the prostate 83, as shown in Fig. 5.

To perform the treatment here, the applicator 2 is inserted into the urethra 81, the reflector 6 is inserted into the rectum 82, the ultrasonic oscillator 3 is rotated about the axis of the applicator 2, and the target site of the prostate in the surroundings region of the urethra 81 is irradiated with ultrasound 85. The prostatic cancer site 80 is irradiated with the ultrasound 85 once per one revolution of the ultrasonic oscillator 3, and the other entire peripheral target sites are also similarly irradiated with the ultrasound 85 once per one revolution. The reflector 6 is moved as indicated by the chain-line while extending the rectum 82, in a manner corresponding to or coordinated with the rotation of the ultrasonic oscillator 3. By such operations, it is possible to perform thermotherapy over the whole perimeter of the prostate while concentrating the ultrasound into the target site. Incidentally, the transition region 83b to which the rectum 82 cannot be extended is not treated.

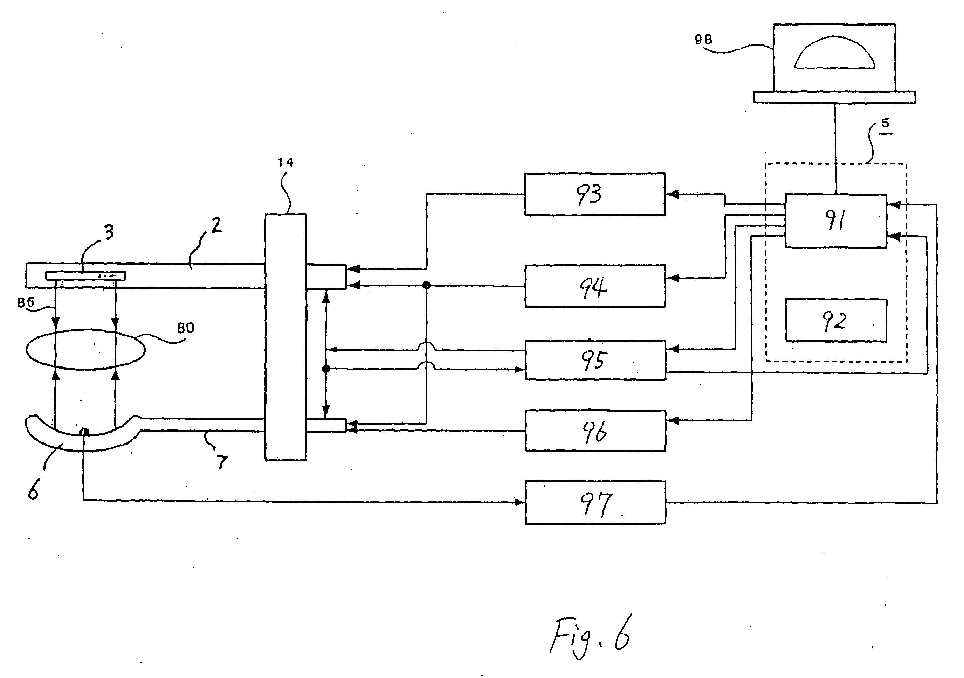

Next, Fig. 6 shows the overall configuration of another example of an energy treatment apparatus 1 which embodies the above-described fundamental aspects. The energy treatment apparatus 1 in the present example, like the ones described above, includes an applicator 2 which is illustrated as being an elongated insertion portion; an energy emission unit 3 disposed in the applicator 2; a reflection means 4 which includes a reflector 6 disposed opposite to the emission unit 3 so as to reflect the energy emitted from the emission unit 3, an operating rod 7; and a controller 5. The applicator 2 and the operating rod 7 of the reflection means 4 are held by an arranging apparatus 14 in an extracorporeal region.

The controller 5 includes a control unit 91 composed of a CPU such as a microprocessor, and a data base 92 storing programs to be executed by the control unit 91 and various kinds of data. Various control mechanism are connected to the control unit 91. These include an emission control means 93 for setting a transmission output and an irradiation pattern for driving the emission unit 3, for example an ultrasonic oscillator, a cooling liquid control means 94 for supplying and draining a cooling liquid to and from the applicator 2 and the reflection means 6, an arrangement control means 95 for setting the distance between and the directions of the applicator 2 and the reflection means 4, a reflection control means 96 for controlling, for example, the radius of curvature of the reflector 6 of the reflection means 4, and an energy detection means 97 for detecting the intensity of the energy, for example ultrasound, emitted from the energy emission unit 3. Further, a monitor 98, which can be of the cathode ray tube or liquid crystal type, is provided as a display means for displaying information, for example the results of arithmetic operations of input information, etc.

As the cooling liquid control means 94, there is provided a means for circulatorily injecting and draining a cooling liquid to prevent surface layers of the living body tissues other than the target site, i.e., surface layers making contact with the applicator 2 and the reflector 6, from being undesirably excessively heated by the ultrasound emitted from the ultrasonic oscillator 3.

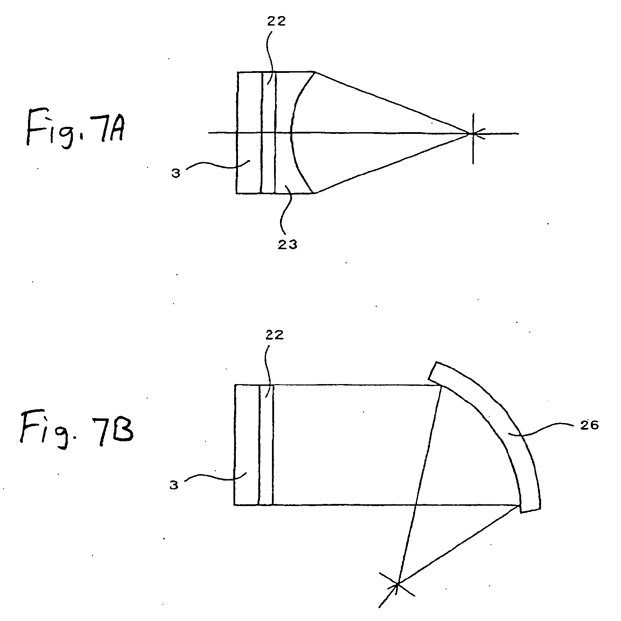

Where the ultrasonic oscillator is used as the emission unit 3, it is desirable to irradiate the target site 80 with the ultrasound while converging the ultrasound. Fig. 7 shows configurations for converging the ultrasound. In the example shown in Fig. 7A, the ultrasonic oscillator 3 is combined with a 1/2 wavelength plate 22, to which an acoustic lens 23 is attached. The ultrasound generated by the ultrasonic oscillator 3 penetrates through the 1/2 wavelength plate 22, and is then converged by the acoustic lens 23 to be radiated or directed to the target site 80. In addition where irradiation with a collimated ultrasound is adopted, the acoustic lens 23 is replaced so as to convert the ultrasound into a collimated ultrasound, whereby the target site 80 can be irradiated with the collimated ultrasound. In another example shown in Fig. 7B, a reflector 26 having a concave surface is arranged in place of the acoustic lens. By use of the concave-surfaced reflector 26, the ultrasound can be converged. Though not shown, it is also possible to irradiate the target site 80 with the ultrasound while diverging the ultrasound by an acoustic lens.

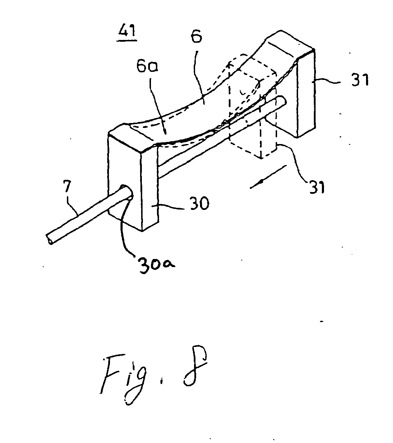

The reflection means 4 can take various forms, a number of which are described below. The reflection means 41 according to the illustrative example shown in Fig. 8 includes a thin strip-shaped reflector 6 for reflecting the radiated ultrasound, with the ends of the reflector 6 being fixed to a respective support base 30, 31 disposed with a predetermined spacing between the bases so that the reflector 6 is curved. An operating rod 7 passes through a through-hole 30a formed in the support base 30 so that the rod can move relative to the base, and the distal end of the operating rod 7 is fixed to the support base 31 so that the rod 7 and the base 31 are axially movable together. The reflection means 41 is configured so that when the operating rod 7 is moved, the curvature of a reflective surface 6a of the paper tablet-shaped reflector 6 is varied. Specifically, when the operating rod 7 is pulled toward the viewer's side, the support base 31 is moved to the illustrated broken-line position, whereby the curving of the reflector 6 is augmented and the radius of curvature of the reflective surface 6a is reduced. On the other hand, when the operating rod 7 is pushed, the radius of curvature of the reflector 6 is enlarged. Therefore, for example where it is desired to appropriately reflect the radiated ultrasound toward the site to be treated (target site), the operating rod 7 is pulled or pushed to change the curvature of the reflector 6 and ensure that the target site 80 is irradiated with the reflected ultrasound.

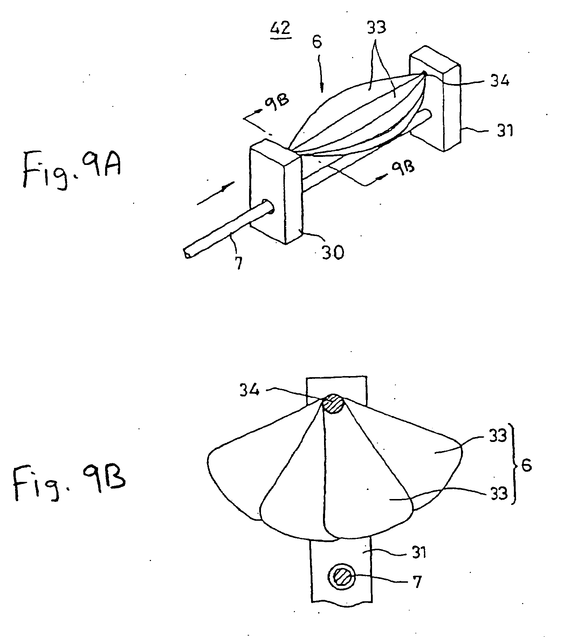

A reflection means 42 according to an illustrative example shown in Figs. 9A and 9B includes a reflector 6 composed of a plurality of reflector elements 33, each of which has a width largest at the center thereof and gradually decreasing toward both ends thereof, and which has both sides formed in an arch shape. The plurality of reflector elements 33 are so arranged as to be bundled into a hemispherical shape, and both ends thereof are supported on support bases 30, 31 in such a manner that integral shafts 34 pass therethrough. The other aspects of the reflector 6 are similar to those described above in connection with the Fig. 8 version, where an operating rod 7 passes through a hole in one of the bases 30 and has a distal end fixed to the other base 31. The reflection means 42 is configured so that the curvature of a reflective surface 6a of the reflector 6 can be varied by operating the operating rod 7.
Specifically, when the operating rod 7 is pushed, the distance between the support bases 30, 31 is enlarged and the overall width of the reflector 6 is reduced, and the radius of curvature of the reflective surface 6a is enlarged. On the other hand, by pulling the operating rod 7, the distance between the support bases 30, 31 is shortened, the overall width of the reflector 6 is enlarged, and the radius of curvature of the reflective surface 6a is reduced.

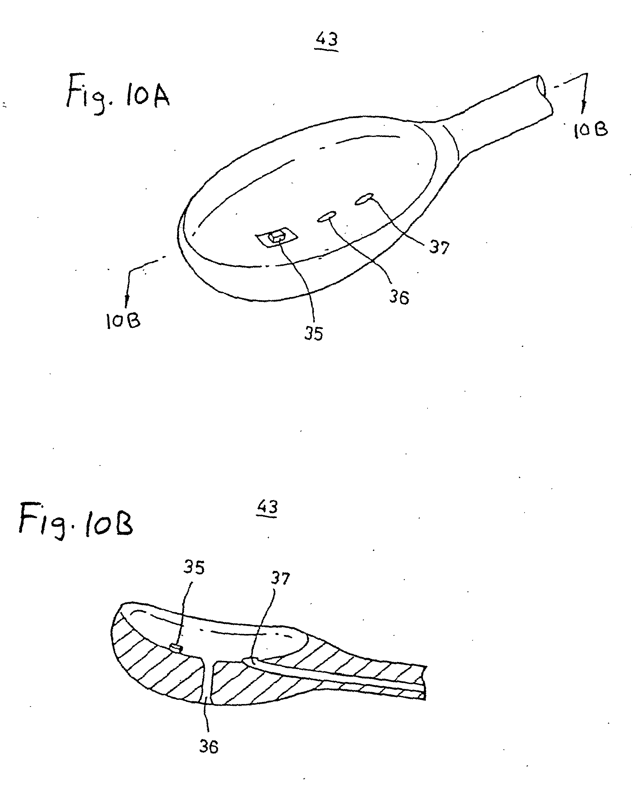

A reflection means 43 according to another embodiment shown in Figs. 10A and 10B includes a spoon-shaped reflector 6 formed at the distal end of an operating rod 7. In this reflection means 43, an ultrasound sensor 35, for example, is provided at the center of the reflector 6 and is adapted to detect the ultrasound emitted from the emission unit 3. Other portions of the reflector 6 are provided with an air hole 36 for discharging air present in the inside of the spoon shape reflector and with an ultrasound jelly injection hole 37 for injecting an ultrasound jelly. In this case, the ultrasound jelly injection hole 37 is formed to communicate with the reflective surface of the spoon shape reflector through the operating rod 7. In the use of the reflection means 43, where, for example, ultrasound is emitted from the emission unit 3, the output intensity of the ultrasound is detected by the ultrasound sensor 35 provided in the reflection means 43. Output from the ultrasound sensor 35 is fed back to a control unit 91, whereby the ultrasound output of the emission unit 3 can be controlled. In addition, with ultrasound jelly injected through the ultrasound jelly injection hole 37 into the inside of the spoon-shaped reflector 6, air present between the reflective surface 6a and a living body wall is discharged through the air hole 36. If air is present, the propagation of ultrasound could be hampered. Since air is removed by the injection of the ultrasound jelly, the ultrasound emitted from the emission unit 3 favorably reaches the reflective surface.

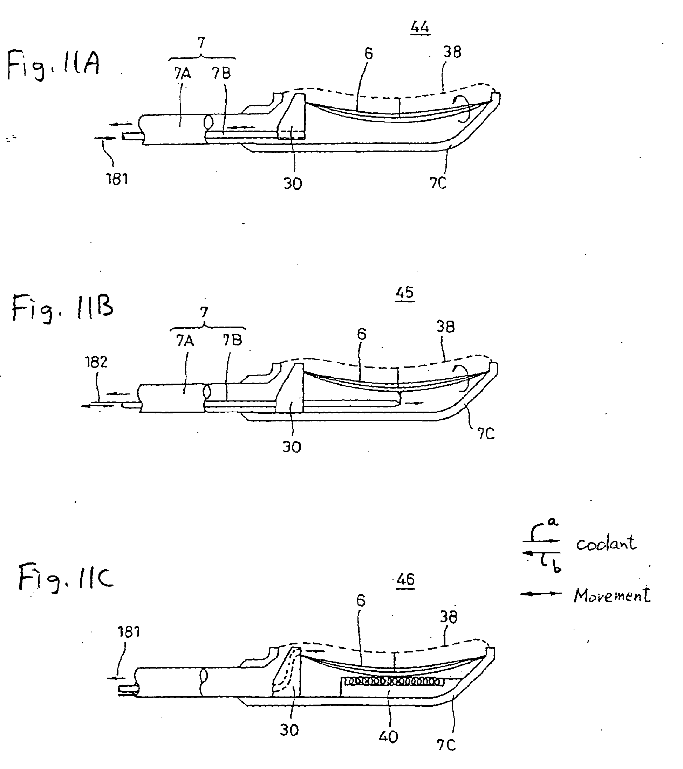

Figs. 11A to 11C show further embodiments of the reflection means. These embodiments pertain to situations in which a cooling liquid, for example cooling water, is injected into the reflector 6 so as to moderate the heating of sites other than the target site, i.e., the surface layer portions of the living body tissue making contact with the reflection means.

The reflection means 44 shown in Fig. 11A is provided with an operating rod 7 comprised of a tubular outer operating rod 7A and a pipe-shaped inner operating rod 7B movable in the axial direction of the outer operating rod 7A, and a reflector 6 composed of a plurality of reflector elements similar to those shown in Figs. 9A and 9B. The reflector 6 is disposed inside a dish-shaped portion 7C provided on the distal end side of the outer operating rod 7A. The reflector 6 is fixed at its one end to the dish-shaped portion 7C and at its other end to a support base 30 which is attached to the distal end of the inner operating rod 7B so that the support base 30 is movable in the axial direction together with the inner operating rod 7B. In this reflection means 44, the curvature of the reflector 6 can be changed, as described above in connection with Figs. 9A and 9B by advancing or retracting the inner operating rod 7B. The surrounding dish-shaped portion 7C and the reflector 6 disposed therein are covered with a flexible film 38 so that the inside of the dish-shaped portion 7C is sealed in a liquid-tight manner. The flexible film 38 is formed of a material which does not block the ultrasound, for example, silicone rubber or latex. The reflection means 44 can also be provided with a cooling means such that a cooling liquid (e.g., cooling water) 181 is injected through the pipe-shaped inner operating rod 7B into the reflector 6 (arrow a) to cool the region where the reflector 6 is disposed, and thereafter the cooling liquid 181 is discharged through the tubular outer operating rod 7A to the exterior (arrow b). The cooling liquid 181 can be used in a circulatorily fashion, for example. By the circulation of the cooling liquid 181, the whole part of the reflector 6 is cooled, thus making it possible, for example, to remove the heat generated in the vicinity of the reflector 6 when the ultrasound emitted from the emission unit 3 is reflected by the reflector 6, and to thermally treat the target site in a generally concentrated manner.

Another embodiment of a reflection means 45 is shown in Fig. 11B and comprises a pipe-shaped inner operating rod 7B passing through a through-hole formed in a movable support base 30 and fixed to the base 30. A wire 182 capable of advancing and retracting inside the inner operating rod 7B is also provided, with the distal end of the wire 182 attached to the center of a reflector 6. Other aspects of this version are similar to those described above in connection with the embodiment shown in Fig. 11A and so corresponding features are identified by the same reference characters as used above and a description of such features is not repeated. In this reflection means 45, by advancing and retracting the wire 182, the central portion of the reflector 6 is moved up and down in the illustration so that the curvature of the reflector 6 can be changed by advancing or retracting the support base 30. Cooling the vicinity of the reflector 6 can be also performed in the same manner as in the Fig. 11A embodiment.

A further reflection means 46 shown in Fig 11C comprises a reflector 6 formed of a bimetallic material, and a heater 40 disposed on the lower side of the reflector 6. Other features of this version are similar to those described above in connection with the embodiment shown in Fig. 11A, and corresponding features are identified by the same reference characters as used above and a description of such features is not repeated. In this reflection means 46, the curvature of the reflector 6 can be changed by curving the reflector 6 through heating by the heater 40. Cooling of the vicinity of the reflector 6 can also be conducted in the same manner as in the version shown in Fig. 11A.

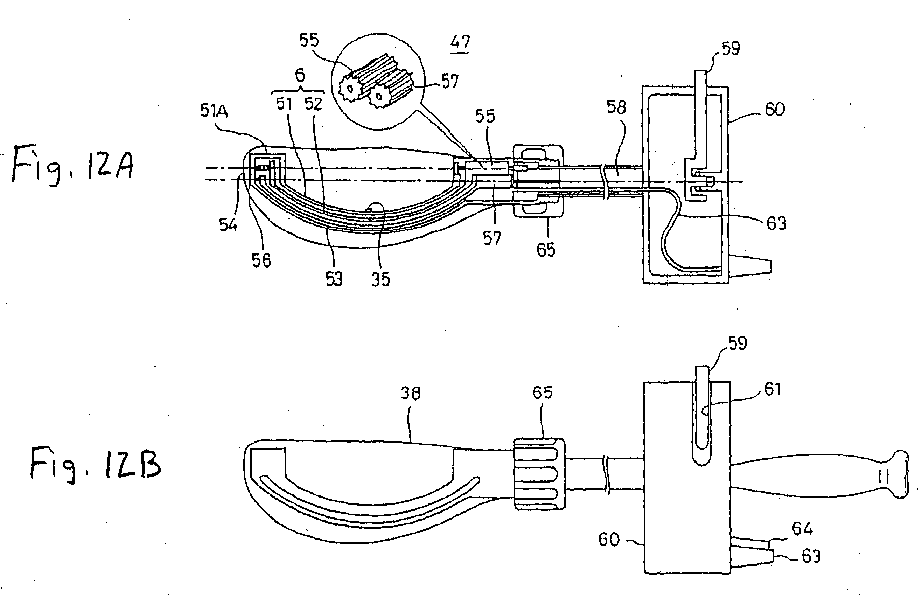

Fig. 12 shows yet another embodiment of the reflection means. In this embodiment, the reflector is narrowed for easier insertion at the time of insertion, and is spread to a required size after the insertion. In the reflection means 47 according to this embodiment, the reflector 6 is comprised of a plurality of reflector elements 51, 52, 53, each of which has a reflective surface with a required curvature and which are overlapped on each other so that they form a reflector with a required size when spread. Specifically, the reflector 6 is provided with a first reflector element 51 having a reflective surface with a required curvature and folded in two so as to be connected at an intermediate position, and second and third reflector elements 52, 53 each of which has a reflective surface with a required curvature and which are overlapped between the folded members of the first reflector element 51.

One end of the second reflector element 52 is rotatably attached to a shaft 54 integral with a folded connection portion 51A of the first reflector element 51, and the other end is attached to the shaft of a first rotatable gear 55 disposed on the same axis as the shaft 54. One end of the third reflector element 53 is rotatably attached to a shaft 56 integral with the folded connection portion 51A of the first reflector element 51, and the other end is attached to the shaft of a second rotatable gear 57 disposed on the same axis as the shaft 56. The first rotatable gear 55 is shaft-supported between an end portion of the first reflector element 51 and a base portion on the side of the operating rod 7. The second rotatable gear 57 is connected to a rotary shaft 58 disposed through the operating rod 7. The first and second rotatable gears 55, 57 mesh with each other. An operating lever 59, which may be operated manually by way of example, is attached to the rotary shaft 58. The operating rod 7 is provided with a housing 60, which is provided with a guide groove 61 for the operating lever 59, and the operating lever 59 is turned along the guide groove 61. Furthermore, a flexible film 38 for covering the whole part of the reflector 6 is provided in the same manner as in the above-described embodiment, and an injection tube 63 for injecting a cooling liquid through the housing 60 into the reflector 6 and a drain tube 64 for draining the cooling liquid are also provided. A portion of the reflection means 47 and the operating rod 7 are connected to each other through a connection means 65.

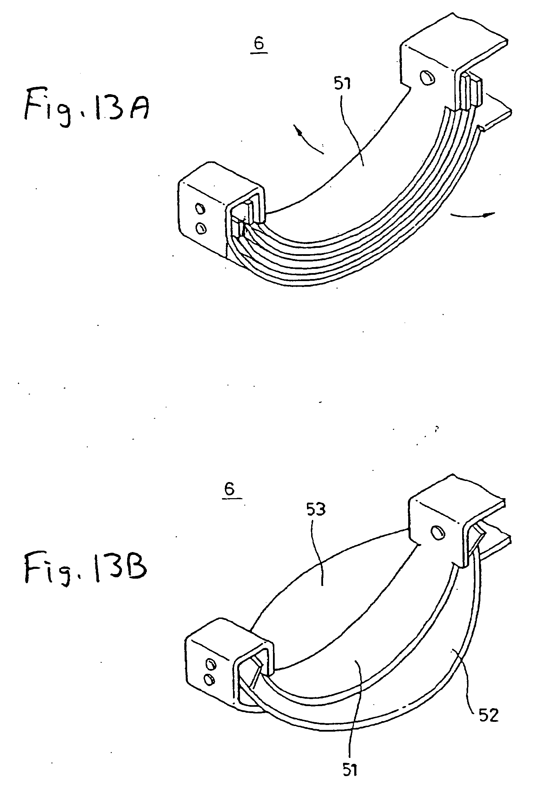

As mentioned above, this reflector 6 includes three reflector elements 51, 52, 53 which are generally folded onto each other or overlapping each other as shown in Fig. 13A at the time of insertion into a body lumen. When the reflector 6 is inserted in a predetermined position inside the body lumen, the rotary shaft 58 can be rotated by turning the operating lever 59, thus causing rotation of the second rotatable gear 57. The first rotatable gear 55 is rotated reversely, whereby the second reflector element 52 and the third reflector element 53 attached respectively to the rotatable gears 55, 57 are spread to the left and the right with the first reflector element 51 as a center. The result is a spoon-shaped reflector 6 with a desired breadth as shown in Fig. 13B.

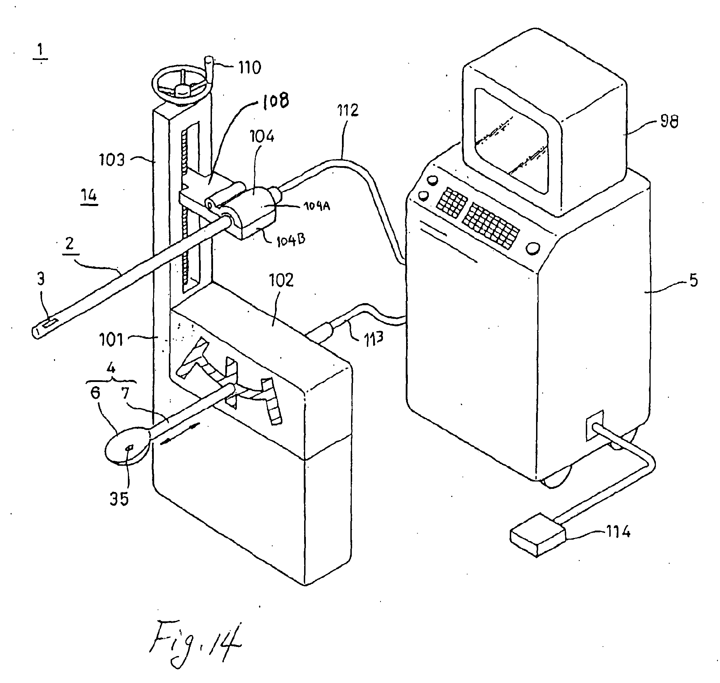

Fig. 14 shows an embodiment of the overall configuration of the energy treatment apparatus according to another illustrative example. This embodiment can be used when applying the energy treatment apparatus to prostatic treatment. The energy treatment apparatus 1 comprises an arranging apparatus for arranging both an applicator 2 comprising an ultrasonic oscillator 3 as the energy emission unit and a reflection means 4 (i.e., an arranging apparatus 14 for holding both the applicator 2 and the reflection means 4), a controller 5 for controlling both the ultrasonic oscillator 3 and the reflection means 4, and a monitor 98.

The arranging apparatus 14 includes a first holding unit 102 for holding the reflection means 4 on a principal surface of a base 101, a second holding unit 104 supported on a support column 103 extending from one side of the base 101 and functioning to hold the applicator 2 at a position opposite to the first holding unit 102, and a position adjusting mechanism for adjusting the positions of the ultrasonic oscillator 3 and the reflection means 4. The first holding unit 102 holds the reflection means 4 so that the position and the direction of a spoon-shaped reflector 6 can be adjusted. For example, as shown in Fig. 5, the reflection means 4 is so held that the reflector 6 can be moved inside the rectum 82 with the prostate 83 as a center and in a manner approaching and moving away from the prostate, and can be rotated with the operating rod 7 as a center. In Fig. 14, the operating rod 7 for the reflector 4 is moved in the region indicated by cross-hatching on the first holding unit 102 so that it is held in a desired position and a rotational position.

The second holding unit 104 is comprised of a cover portion 104A and a fixed portion 104B, in the same manner as the first holding unit 102, so as to hold in a clamping type manner the applicator 2 which is circular in cross-section. The second holding unit 104 is supported on a support member 108, which is vertically movable along the support column 103, and is vertically moved through the support member 108. The vertical movements can be controlled, for example, by a magnetic scale using a magnetic sensor. With this arrangement, the position of the second holding unit 104, and hence the distance between the reflection means 4 and the ultrasonic oscillator 3, can be set by use of the magnetic scale through operation of a handle 110 to move the support member 108 through a feed shaft having a feed screw. The position adjusting mechanism is comprised of means for respectively effecting vertical movements of the second holding unit 104, movement of the applicator 2 in the axial direction and turning of the applicator 2 about the axis in the second holding unit 104, and movement of the reflection means 4 in the axial direction, turning of the reflector about the axis of the operating rod 7 and movement of the operating rod 7 within the hatched region, in the first holding unit 102.

The applicator 2 provided therein with the ultrasonic oscillator 3 is connected to the controller 5 via a cable 112. The cable 112 includes an electric wiring, and cooling liquid feed/drain tubes for injecting and draining a cooling liquid into and from the region where the ultrasonic oscillator 3 is disposed inside the applicator 2. With respect to the reflection means 4, cooling liquid feed/drain tubes 113 for injecting and draining a cooling liquid and a signal wire extending from an output detection sensor are connected to the controller 5. The controller 5 includes a key operating unit, the above-mentioned control unit 91, the data base 92, and cooling liquid tanks and the like. The controller 5 is adapted to effect the control of the output of the ultrasound emitted from the ultrasonic oscillator 3, the control of the flow rates of the cooling liquid, the processing of a detection signal from the ultrasound sensor 35 at the reflector 6, the control of other functions (described later) when the other functions are provided, etc. A footswitch 114 may also be provided.

The energy treatment apparatus according to another illustrative example aims at enhancing the concentration of energy on a target site by use of the basic configuration generally described above. This apparatus can comprise an elongate applicator, an energy emission unit disposed in the applicator, a reflection means disposed at a position opposite to the applicator to reflect the energy emitted from the emission unit, a controller for controlling the energy emission unit and the reflection means, and an arranging apparatus for arranging the applicator and the reflection means, with the energy emitted from the emission unit being collected into the target site by way of different routes at the time of treatment.

According to the energy treatment apparatus in this illustrative example, the energy emission unit and the reflection means are provided and the energy emitted from the emission unit is radiated to the target site by way of different routes, so that it is possible to concentratively irradiate the target site with the energy, without significantly heating the living body tissues other than at the target site. It is possible to heat the target site to a desired or relatively optimum thermotherapeutic temperature, and to perform thermotherapy in a relatively short time.

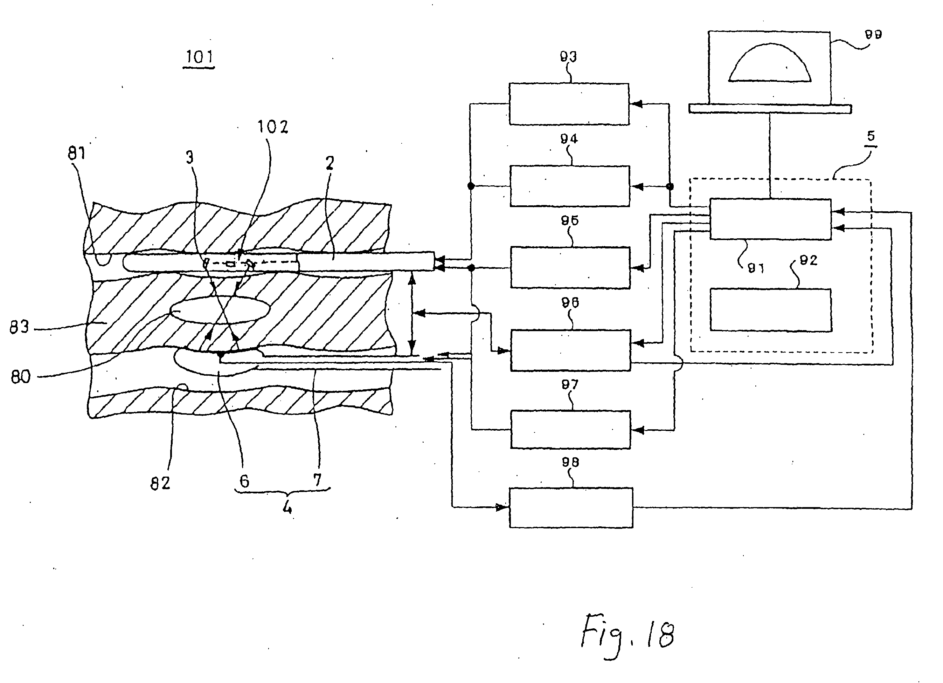

Fig. 18 illustrates an overall configuration of another illustrative example of an energy treatment apparatus in which a moving mechanism for the energy emission unit is added as one mechanism by which the energy emitted from the emission unit is concentrated into the target site by way of different paths.

The energy treatment apparatus 101 shown in the embodiment of Fig. 18, like the above-described embodiments, comprises an applicator 2 which is generally in the form of an elongated insertion portion, a side emission type energy emission unit 3 disposed in the applicator 2, a reflection means 4 comprised of a reflector 6 and an operating rod 7 and disposed at a position opposite to the applicator 2 to reflect the energy emitted from the emission unit 3, a moving mechanism 102 for moving the energy emission unit 3 in the axial direction of the applicator 2, and a controller 5. The applicator 2 and the operating rod 7 of the reflection means 4 are held by an arranging apparatus 14 in an extracorporeal region. The controller 5 includes a control unit 91 composed of a CPU such as a microprocessor, and a data base 92 storing programs to be executed by the control unit 91 and various kinds of data. The control unit 91 is connected to a movement control means 93 for driving the emission unit 3 (for example an ultrasonic oscillator), an emission control means 94 for setting an irradiation pattern, a cooling liquid control means 95 for supplying and draining a cooling liquid to and from the applicator 2 and the reflection means 6, an arrangement control means 96 for setting the distance between and the directions of the applicator 2 and the reflection means 4, a reflection control means 97 for controlling, for example, the radius of curvature of the reflector 6 of the reflection means 4, and an energy detection means 98 for detecting the intensity of the energy, for example, ultrasound, emitted from the energy emission unit 3 are connected to the control unit 91. Further, a monitor 99 of the cathode ray tube type or liquid crystal type, for example, is provided as a display means for displaying infromation including the results of arithmetic operations of input information, etc.

As described previously in connection with the apparatus shown in Fig. 6, the cooling liquid control means 95 can include means for circulatorily injecting and draining a cooling liquid to prevent surface layers of living body tissues other than the target site (i.e., surface layers making contact with the applicator 2 and the reflector 6) from being heated by the ultrasound emitted from the ultrasonic oscillator 3.

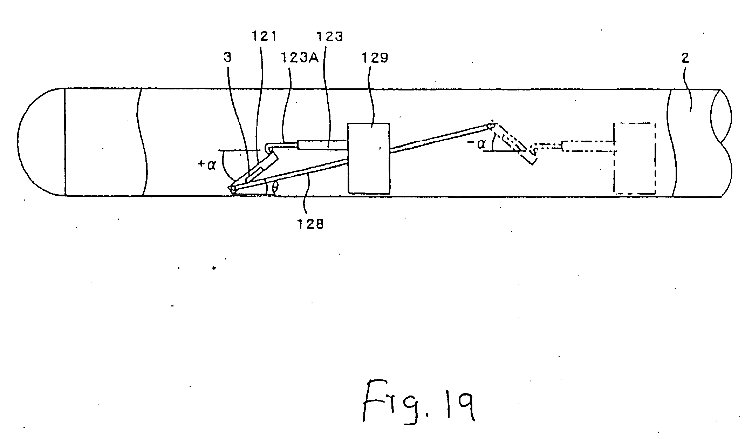

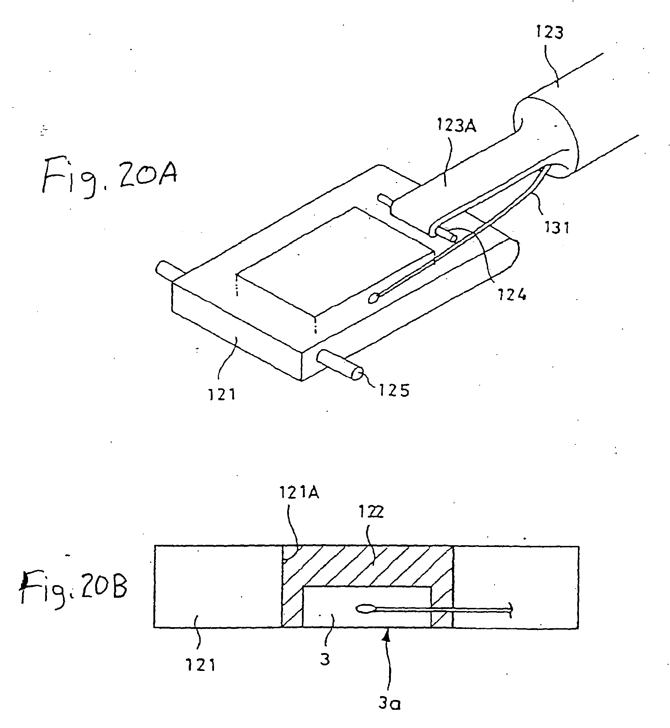

When the ultrasonic oscillator is used as the emission unit 3, the ultrasonic oscillator is integrally arranged in an opening 121A in a base 121, with a cushioning material 122 therebetween as shown in Fig. 20B. In this case, the ultrasonic oscillator 3 is so disposed that the oscillator surface thereof is flush with the lower surface of the base 121. The upper surface of the base 121, with the ultrasonic oscillator 3 integrally provided therein, is mounted to a distal end portion 123A of one arm 123 via a shaft 124 so as to be turnable relative to the distal end portion 123A as shown in Fig. 20A. The proximal portion side of the arm 123 is attached to a slide portion 129 of a drive unit so that the arm 123 is reciprocated in the axial direction of the applicator 2 by sliding of the slide portion 129 as generally shown in Fig. 19. In connection with the moving means for moving the arm 123 (e.g., the drive unit comprising the slide portion 129), it is possible to provide a regulation means by which the movement amount and the moving speed of the reciprocating movement of the ultrasound emission unit can be regulated. As shown in Fig. 20A, a required driving signal is applied to the ultrasonic oscillator 3 through a wiring 131, thereby causing ultrasonic oscillation.

The base 121 is integrally provided with engagement pins 125 on both side surfaces of the distal end of the base 121 on the side opposite to the side of attachment to the arm 123. The engagement pins 125 are engaged respectively in engagement guide grooves 128 formed in a pair of guide plates 127 disposed inside the applicator 2. The guide grooves 128 are formed to be inclined at an angle θ moving from the distal end side toward the proximal end side of the applicator 2 along the axial direction of the arm 123. The base 121 integral with the ultrasonic oscillator 3 is moved along the guide grooves 128, with which the engagement pins 125 are engaged, as the arm 123 is moved. This causes the base 121 to be turned with the shaft 124 as a center. In this way, the angle α of an ultrasound emission surface 3a is varied. Specifically, a setting is made such that when the engagement pins 125 of the base 121 are located at the lower ends of the guide grooves 128, the ultrasound emission surface 3a is set at a predetermined angle +a , then the ultrasound emission surface 3a becomes parallel to the axis of the arm 123 (angle a = 0°) when the engagement pins 125 are at intermediate position between the upper ends and the lower ends of the guide grooves 128, and thereafter the ultrasound emission surface 3a is set at an angle -α against the axis of the arm 123 when the engagement pins 125 are located at the upper ends of the guide grooves 128.

It is desirable that the normal living body tissues other than the target site, for example, the surface layer portions making contact with the applicator 2, are not significantly heated at the time of treatment. To achieve this result, the applicator 2 may be so configured that the arm 123 connected to the base 121 which is integral with the ultrasonic oscillator 3 has a pipe structure so that a cooling liquid can be injected into the applicator 2, particularly into the vicinity of the ultrasonic oscillator 3, through the arm 123. The cooling liquid can also be drained from the applicator 2 through a drain tube.

It is also desirable that the ultrasound emitted from the ultrasonic oscillator 3 is radiated in a converging manner to the target site 80. For this purpose, and in a manner similar to that described above in connection with Figs. 7A and 7B, a converging means for converging the ultrasound may be disposed at an intermediate position in the emission path of the ultrasound. Also, as described previously, the ultrasound can be radiated to the target site 80 while being diverged by an acoustic lens.

Referring to Figs. 18, 19, 21 A, 21 B, set forth below is a description describing use of the apparatus in a situation in which the energy treatment apparatus 101 is applied to the treatment of prostatic cancer or BPH.

First, the applicator 2 is inserted into the urethra 81 so that the ultrasonic oscillator 3, which is the emission unit provided at a distal end portion of the applicator 2, is located in the vicinity of the target site 80 to be irradiated. In addition, the reflector 6 is inserted into the rectum 82 through the use of the operating rod 7 and is located in the vicinity of the target site 80 at a position opposite to the ultrasonic oscillator 3.

Next, while driving the slide portion 129 of the drive unit constituting the movement control means 93 and thereby moving the ultrasonic oscillator 3 from a distal end portion of the applicator 2 toward a proximal end portion, the target site 80 is irradiated with the ultrasound emitted from the ultrasonic oscillator 3. A part of the ultrasound radiated to the target site 80 penetrates through the target site 80, is reflected by the reflector 6, and is again radiated to the target site 80. In the present embodiment, attendant on the movement of the base 121 integral with the ultrasonic oscillator 3 in the axial direction of the applicator 2 (i.e., the movement of the base 121 from the distal end side toward the proximal end side of the applicator 2), the engagement pins 125 are guided along the guide grooves 128. The ultrasonic oscillator 3 is thus turned so that the angle between the ultrasound emission surface 3a and the axis of the applicator 2 is changed from +α through 0 to -α, in the same manner as discussed above. The ultrasound from each turned position of the ultrasonic oscillator 3 is necessarily radiated to the target site 80. Therefore, only the target site 80 is always irradiated with the ultrasound in a concentrated manner, whereas body tissues other than the target site 80, for example surface layer portions, are relatively little heated. It is thus possible to heat only the target site 80. It is also possible to perform a high-temperature treatment by repeatedly reciprocating the ultrasonic oscillator 3 in the axial direction of the applicator 2 in this manner.

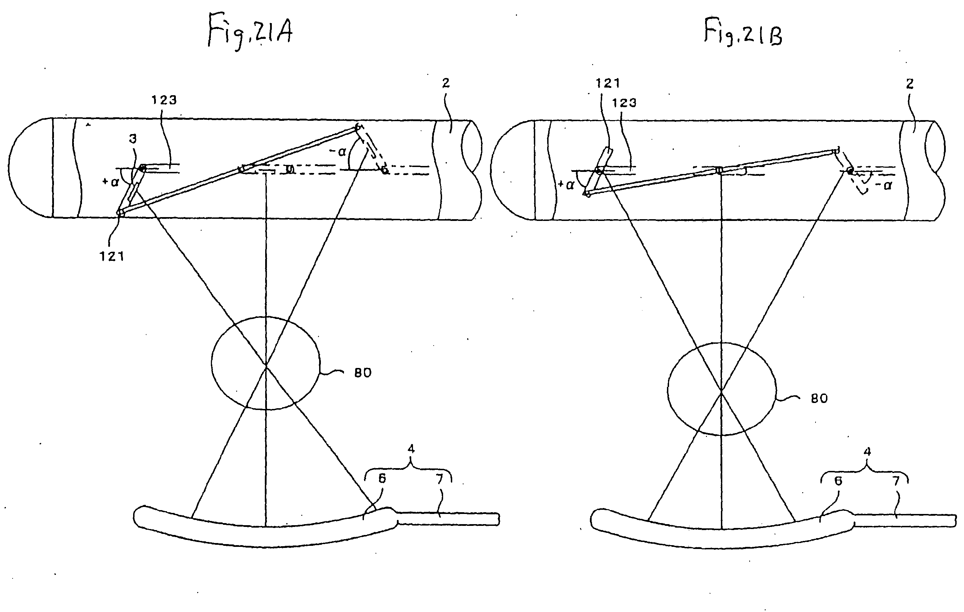

In the high-temperature treatment as described above, the emission unit shown in Fig. 19 is used, and the high-temperature treatment can be performed while changing the angle a between the emission unit and the axis of the applicator as shown in Fig. 21 A. Incidentally, also in the case where the arm 123 is attached to the center of the base 121 as in the emission unit shown in Fig. 21B, it is possible to perform the high-temperature treatment while changing the angle α in the same manner as in the case of Fig. 21A.

Here, before ultrasound treatment, the direction of the emission unit 3 and the distance between the applicator 2 and the reflector 6 are set in a distance/direction setting unit 96. Also, as described above, the intensity of the ultrasound emitted from the emission unit 3 can be detected by an ultrasound sensor 35 constituting the ultrasound detection unit 98 provided at the reflector 6, with the detected intensity being fed back to the control unit 91, thereby regulating the ultrasound output of the emission unit 3. Furthermore, at the time of the ultrasound treatment, cooling liquid can be supplied through the cooling liquid control means 95 into the applicator 2 and into the reflector 6 as required.

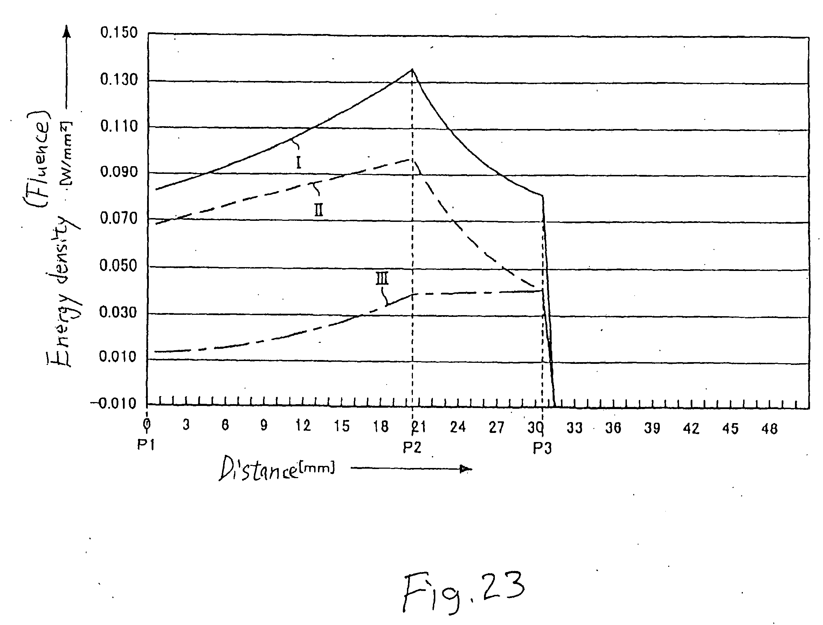

Fig. 23 is a graph showing the results of calculation of the accumulation of ultrasound by the energy treatment apparatus 101 described above. The ordinate (y-axis) indicates energy density (fluence: W/cm²), and the abscissa (x-axis) indicates the distance (mm) between the ultrasonic oscillator 3 and the reflector 6.

The graph in Fig. 23 represents the ultrasonic energy density at the target site P2 in the case where the ultrasonic oscillator 3 is disposed at position P1 (= 0 mm), the target site 80 is present at position P2 (= 20.5 mm), and the reflector 6 is disposed at position P3 (= 30.5 mm). The conditions of ultrasound irradiation are as given in Table 1.

[Table 1]

| | |
|---|---|
| Attenuation coefficient | -1.5 dB/cm |
| Moving distance of emission unit | 30 mm |
| Condensing distance (distance from emission unit to target site) | 20.5 mm |
| Position of reflector (distance from emission unit) | 30.5 mm |
| Ultrasound emission power | 20 W |
| Spreading angle of ultrasound | 10 deg |
| Ultrasound emission beam diameter | 8 mm |

Curve II in Fig. 23 represents the ultrasonic energy density distribution when the ultrasound is emitted from the ultrasonic oscillator 3 toward the target site P2. Curve III represents the ultrasonic energy density distribution after a part of the ultrasound penetrates through the target site and is reflected by the reflector 6. Curve I represents the ultrasonic energy density distribution in the condition where the ultrasonic energy density of curve II and the ultrasonic energy density of curve III arc added to each other.

From curve I, it is clearly recognized that the ultrasound emitted from the ultrasonic oscillator 3 and the ultrasound reflected from the reflector 6 are superposed on each other at the target site P2, whereby the concentration of the ultrasonic energy is enhanced, and the ultrasonic energy density is gradually lowered as the distance from the target site P2 increases.

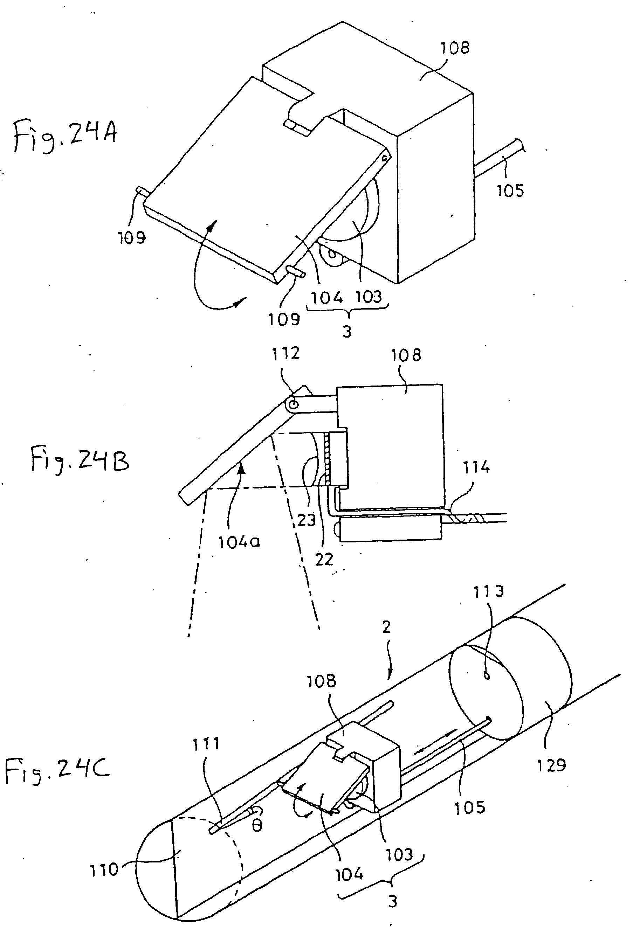

Figs. 24A-24C show other illustrative examples of the emission unit 3 for concentrating the ultrasound into or at the target site 80 by moving the emission unit 3 in the axial direction of the applicator 2. The emission unit 3 here, for example in the case of ultrasound treatment, is comprised of an ultrasonic oscillator 103 and an ultrasonic reflector such as a movable mirror 104 for directing the ultrasound emitted from the ultrasonic oscillator 103 toward the target site 80.

Where an ultrasound emission unit is used as the emission unit 3, the ultrasonic oscillator 103 is connected to a slide portion 129 of a drive unit through an arm 105 extending in the axial direction of the applicator 2. The ultrasonic oscillator 103 is reciprocated in the axial direction of the applicator 2 by the slide portion 129. The ultrasonic oscillator 103 is supported on the front surface side of a support portion 108, and the support portion 108 is fixed to the distal end of the arm 105.

On the other hand, the upper end of the mirror 104 is movably mounted to the upper end on the front surface side of the support portion 108 through a shaft 112. Engagement pins 109 projecting from both side portions of the lower end of the mirror 104 are engaged with guide grooves 111 formed in a pair of guide plates 110 disposed inside the applicator 2. The guide grooves 111 are formed so as to be inclined at a required angle θ during movement from the distal end side toward the proximal end side of the applicator 2 along the axial direction of the arm 105. With the arm 105 moved, the engagement pins 109 are moved along the guide grooves 111 and so the angle of the mirror surface of the mirror 104 can be varied with the shaft 112 as a center.

It is desirable that normal body tissues other than the target site 80, for example surface layer portions, making contact with the applicator 2 are not significanty heated at the time of treatment. To achieve this result, the applicator 2 may be configured so that the arm 105 connected to the support portion 108 for the ultrasonic oscillator 103 is generally configured as a pipe or hollow member allowing a cooling liquid to be injected through the arm 105 into the applicator 2, particularly into the vicinity of the emission unit 3, with the cooling liquid being drained through a drain hole 113 formed in the slide portion 129.

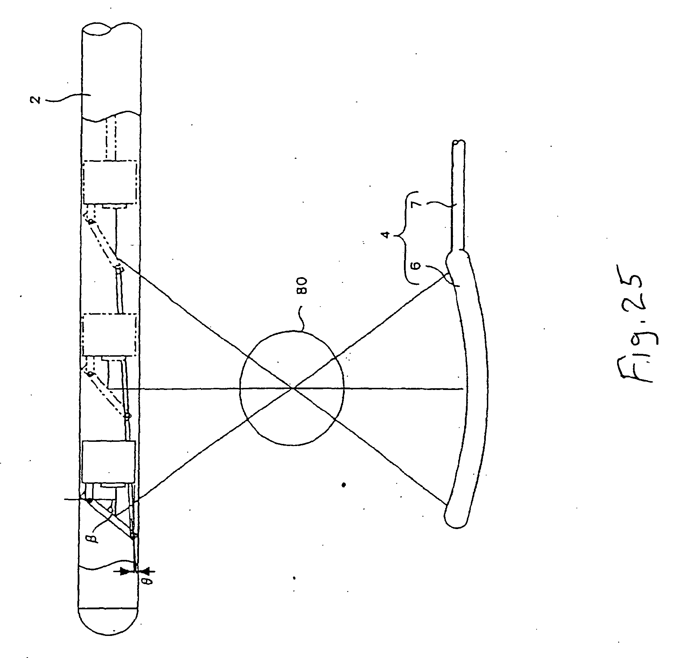

The ultrasound emitted from the ultrasonic oscillator 103 is reflected by the mirror surface 104a of the movable mirror 104 to be radiated to the target site 80. In this case, an acoustic lens for converging the ultrasound may be attached to the ultrasound emission surface of the ultrasonic oscillator 103, with a 1/2 wavelength plate 22 therebetween in the same manner as above-described. As shown in Fig. 24B, a required drive signal can be sent to the ultrasonic oscillator 103 through a wiring 114, thereby achieving ultrasonic oscillation. Referring to Fig. 25, the operation of the emission unit 3 shown in Figs. 24A-24B is as follows. While driving the slide portion 129 of the drive unit constituting the movement control means 93 and thereby moving the emission unit 3 from the distal end portion of the applicator 2 toward the proximal end portion, the target site 80 is irradiated with the ultrasound emitted from the ultrasonic oscillator 103. A part of the ultrasound radiated to the target site 80 penetrates or passes through the target site 80, is then reflected by the reflector 6, and is again radiated to the target site 80. The target site 80 is thus irradiated with both the reflected ultrasound reflected from the reflector 6 and the next emitted ultrasound in the same manner as described above. In this embodiment, based on the movement of the ultrasonic oscillator 103 in the axial direction of the applicator 2, the engagement pins 109 of the mirror 104 are guided along the guide grooves 111, whereby the mirror 104 is turned so that the angle β between the mirror 104 and the ultrasonic oscillator 103 is increased. The ultrasound emitted from each turned position of the mirror 104 is necessarily radiated to the target site 80. Therefore, only the target site 80 is always irradiated with the ultrasound in a concentrated manner, and living body tissues other than the target site 80, for example surface layer portions, are heated only momentarily. It is thus possible once again to heat only the target site 80.

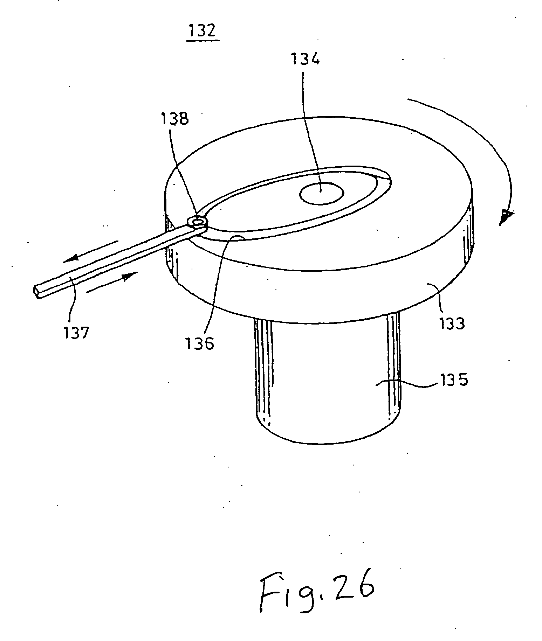

Fig. 26 shows an example of the mechanism associated with the drive unit, for reciprocating the emission unit. The drive unit 132 is provided with a direct driven follower-type grooved cam 133, and the rotary shaft 134 of the grooved cam 133 is connected to the shaft of a motor 135 constituting an electric drive means. The grooved cam 133 is provided with an eccentric elliptic groove 136, and a cam follower 138 provided at the proximal end of a rod 137 which is connected to the proximal end of an arm connected to the emission unit 3 is slidably fitted in the groove 136.

Through rotation of the motor 135, the grooved cam 133 is rotated about the rotary shaft 134. In this case, the cam follower 138 is not rotated but slides in the groove 136. Since the rotary shaft 134 is eccentric relative to the groove 136, the rotation thereof causes the rod 137 (and the arm) to repeatedly undergo a translational movement (reciprocating movement).

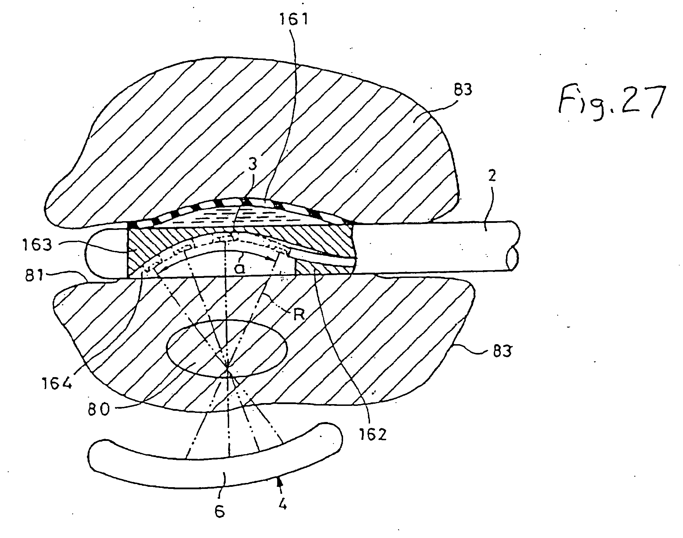

Fig. 27 shows another illustrative example of the emission unit 3 for concentrating the ultrasound into the target site 80 by moving the emission unit 3 along the axis of the applicator 2. This embodiment of the energy treatment apparatus includes an applicator 2 having an ultrasound emission unit 3 and a reflection means 4 comprising a reflector 6, in the same manner as above-described.

The applicator 2 is inserted into the urethra 81, and, when the ultrasound emission unit 3 is located opposite to a target site 80 of the prostate 83, a one-sided balloon 161, which can be positioned at the opposite side relative to the emission unit 3 as illustrated, is expanded to bring the balloon into close contact with the upper surface of the urethra 81 in Fig. 27. This thus better stabilizes the position of the emission unit 3.

The ultrasound emission unit 3 is reciprocated with the range indicated by arrows a as a stroke length. It is necessary for the emission unit 3 to be located on the distal end side relative to a first guide portion 162 when the emission unit is located on the most proximal side. In addition, it is desirable that when the emission unit 3 is located on the most distal side, the emission unit 3 does not exceed the distal end of a second guide portion 163 and the ultrasound passes through an opening 164.

The ultrasound is emitted to a lateral side relative to the longitudinal axis of the emission unit 3 (preferably at a right angle to the longitudinal axis of the emission unit) for an efficient irradiation. Here, the longitudinal axis of the emission unit 3 is always in the direction of a tangent to a circular arc drawn by the second guide portion 163. Therefore, the ultrasound irradiation direction is always directed substantially toward the center (the target site 80) of the circle including the circular arc. Therefore, when the target site 80 is irradiated with the ultrasound during reciprocation of the emission unit 3, the ultrasound emission position is always varied on the surface of the living body tissue in contact with the applicator 2, so that the irradiation time for the surface is short, and the quantity of heat generated there is accordingly relatively small. Additionally, the ultrasound is concentrated at the target site 80 located in the depth of the body tissue, so that only the depth portion can be thermally treated while preserving the surface of the body tissue.

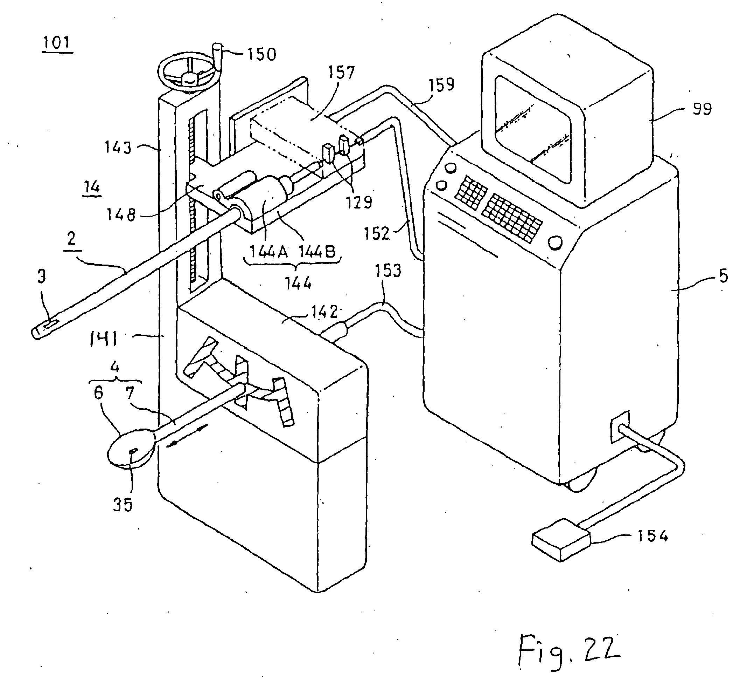

Fig. 22 shows an overall configuration of another illustrative example of an energy treatment apparatus which can be used to emit energy to the target site in such a way that, for example, the emitted energy is collected at the target site by way of different routes. This embodiment of the apparatus, which is similar in many respects to the embodiment of the apparatus described above and illustrated in Fig. 14, is described by way of example in the context of being applied to prostatic treatment. The energy treatment apparatus 101 includes an arranging apparatus for arranging both an applicator 2 having an ultrasonic oscillator 3 as the energy emission unit and a reflection means 4 (i.e., an arranging apparatus 14 for holding both the applicator 2 and the reflection means 4), a controller 5 for controlling both the ultrasonic oscillator 3 and the reflection means 4, and a monitor 99.

The arranging apparatus 14 includes a first holding unit 142 for holding the reflection means 4 on a principal surface of a base 141, a second holding unit 144 supported on a support column 143 extending from one side of the base 141 and functioning to hold the applicator 2 oppositely to the first holding unit 142, and a position adjusting mechanism for adjusting the positions of the ultrasonic oscillator 3 and the reflection means 4. The first holding unit 142 holds the reflection means 4 so that the position and the direction of a spoon-shaped reflector 6 can be adjusted arbitrarily. For example, an operating rod 7 for the reflector 4 is adapted to be moved in the region indicated by the hatching on the first holding unit 142 so that it is held in a variety of desired positions while also be capable of being held at a rotational position.

The second holding unit 144 includes a cover portion 144A and a fixed portion 144B, in a somewhat similar manner to the first holding unit 142, so as to hold the applicator 2 which is circular in cross-section in a clamp type manner. The second holding unit 144 is supported on a support member 148 vertically movable along the support column 143, and is vertically moved through the support member 148. The vertical movements can be controlled, for example, by a magnetic scale using a magnetic sensor. With this configuration, the position of the second holding unit 144, and hence the distance between the reflection means 4 and the ultrasonic oscillator 3, can be set by use of the magnetic scale through operation of a handle 150 to move the support member 148 through a feed shaft having a feed screw. The position adjusting mechanism includes means for respectively effecting the vertical movements of the support member 148, the movement of the applicator 2 in the axial direction and the turning of the applicator 2 about its axis in the second holding unit 144, and the movement of the reflection means 4, the turning of the reflector about the axis of the operating rod 7 and the movements of the operating rod 7 within the hatched region in the first holding unit 142.

Further, a drive unit 157 as the movement control means 93 is disposed on the support member 148 and is adapted to axially move the arm connected to the emission means 3 in the applicator 2 in the axial direction. In the illustrated embodiment, the arm is attached to a slide portion 129 of the drive unit 157. The slide portion 129 of the drive unit 157 is driven by an electric signal supplied from a controller 5 through a cable 159. Thus, the arm, and hence the emission unit 3, can be moved in the axial direction.

The applicator 2 provided with the ultrasonic oscillator 3 is connected to the controller 5 via a cable 152. The cable 152 can be configured to includes electric wiring, and possibly also cooling liquid feed/drain tubes for injecting and draining cooling liquid into and from the region where the ultrasonic oscillator 3 is disposed inside the applicator 2. With respect to the reflection means 4, cooling liquid feed/drain tubes 153 for injecting and draining cooling liquid for the refelction means,and a signal wire extending from an output detection sensor for the reflection means are connected to the controller 5. The controller 5 includes a key operating unit, the above-mentioned control unit 91, the data base 92, and cooling liquid tanks and the like. The controller 5 is adapted to perform the control of the output of the ultrasound emitted from the ultrasonic oscillator 3, the control of the flow rates of the cooling liquid, the processing of a detection signal from the ultrasound sensor 35 at the reflector 6, the control of other functions (described later) when the other functions are provided, etc. A footswitch 154 may also be provided.

According to an aspect of the present invention, an energy treatment apparatus includes a plurality of energy emission units for emitting different-frequency energies, reflection means disposed at a position opposite to the energy emission units to reflect each of the energies emitted from the energy emission units toward a target site, and an arranging apparatus for arranging the energy emission units and the reflection means so that the different-frequency energies respectively form standing waves between the energy emission units and the reflection means, with the target site of a living body tissue being irradiated in a concentrated manner with the energies through superposition of the different-frequency standing waves. The resultant energy obtained through superposition of the plurality of standing waves forms an energy intensity distribution such that the energy intensity is greatest at the target site and decreases as the distance from the target site increases.

An energy treatment apparatus according to this aspect comprises the plurality of energy emission units and the reflection means, with the target site being irradiated with the resultant energy of the standing waves of the plurality of different-frequency energies emitted from the plurality of energy emission units, and with the intensity of the resultant energy being maximized at the target site. Therefore, it is possible to irradiate the target site in a concentrated manner with the energies, without significantly heating living body tissues other than the target site. Thus, it is possible to heat the target site to a relatively optimum temperature for high-temperature treatment.

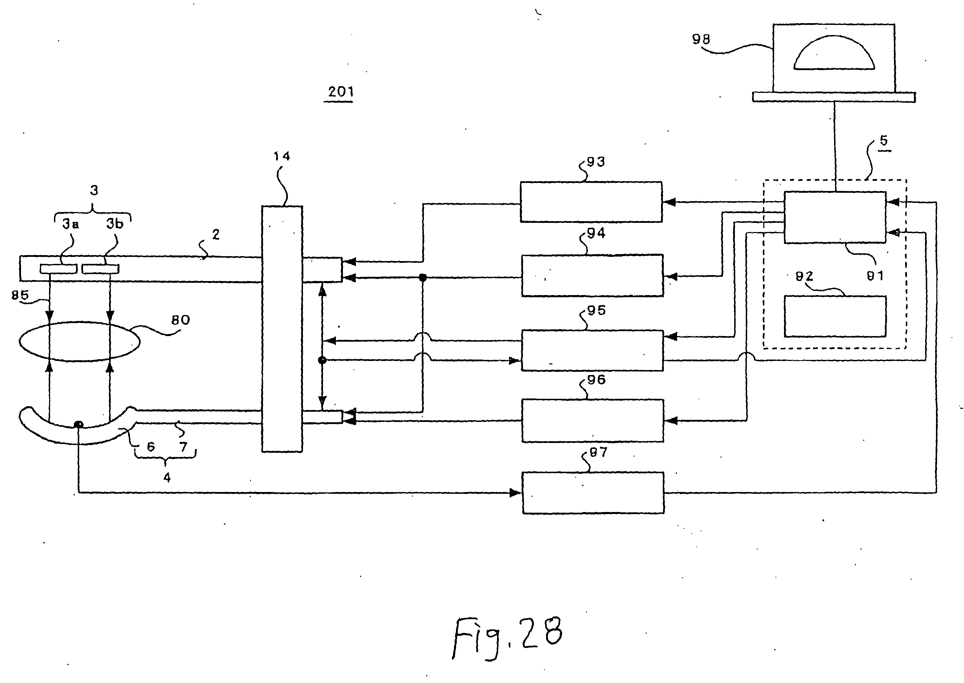

A first embodiment of an energy treatment apparatus according to the present invention will be described with reference to Fig. 28. The energy treatment apparatus 201 according to the present embodiment, which is similar in various respects to other versions of the apparatus described above, includes an applicator 2 which is an elongated insertion portion, an energy emission unit 3 disposed in the applicator 2, a reflection means 4 composed of a reflector 6 and an operating rod 7 which is disposed opposite to the emission unit 3 to reflect the energies emitted from the emission unit 3, and a controller 5. The applicator 2 and the operating rod 7 of the reflection means 4 are held by an arranging apparatus 14 in an extracorporeal region.

The controller 5 comprises a control unit 91 composed of a CPU such as a microprocessor, and a data base 92 storing programs to be executed by the control unit 91 and various kinds of data. A number of control devices are connected to the control unit 91. These include an emission control means 93 for setting transmission outputs and an irradiation pattern for driving the emission unit 3 which in this embodiment is comprised of several emission units 3a, 3b, for example ultrasonic oscillators, a cooling liquid control means 94 for supplying and draining a cooling liquid to and from the applicator 2 and the reflection means 4, an arrangement control means 95 for setting the distance between and the directions of the applicator 2 and the reflection means 4, a reflection control means 96 for controlling, for example, the radius of curvature of the reflector 6 of the reflection means 4, and an energy detection means 97 for detecting the intensities of the energies, for example ultrasounds, emitted from the energy emission units 3, Further, a monitor 98, which can for example be of the cathode ray tube or liquid crystal type, is provided as a display means for displaying the results of arithmetic operations of input information, etc.

The cooling liquid control means 94 is configured to inject and drain in a circulatory fashion a cooling liquid so prevent surface layers of living body tissues other than the target site (i.e., surface layers making contact with the applicator 2 and the reflector 6) from being significantly heated by the ultrasounds emitted from the ultrasonic oscillators 3.

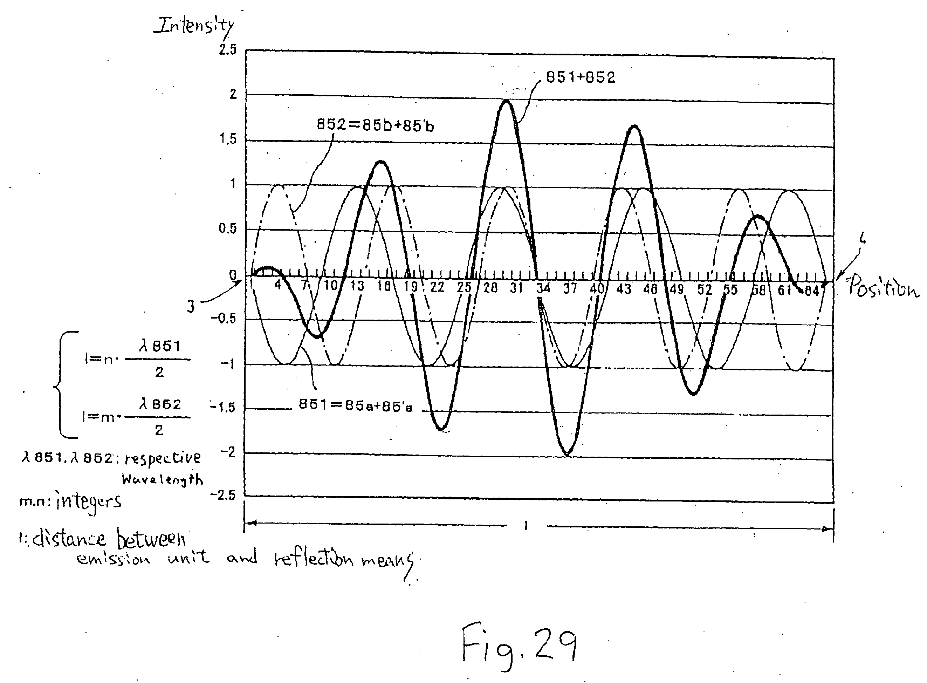

The energy emission unit 3 is comprised of several emission units 3a, 3b, in the disclosed embodiment two ultrasonic oscillators, for emitting different-frequedcy energies. The reflector 6 can be comprised of a common reflector 6 for reflecting the ultrasounds emitted from the two ultrasonic oscillators 3a, 3b. The wavelengths of the respective energies radiated from the two ultrasonic oscillators 3a, 3b are so set that an integer times one half of the wavelength of each of the respective standing waves 851, 852 are equal to the distance 1 between the emission unit 3 and the reflector 6, whereby the standing waves 851, 852 can be produced as seen in Fig. 29.

An irradiation pattern designated by the controller 5 is such that, as shown in Fig. 29, the target site 80 is irradiated with the ultrasounds 85a, 85b emitted from the ultrasonic oscillators 3a, 3b, parts of the emitted ultrasounds 85a, 85b penetrate through the target site 80 and are reflected by the reflector 6 to form reflected ultrasounds 85a', 85b', and the emitted ultrasounds 85a, 85b are superposed on the reflected ultrasounds 85a', 85b' to form a first standing wave 851 and a second standing wave 852. Further, the irradiation pattern is such that the first standing wave 851 and the standing wave 852 are superposed on each other to strengthen each other at the target site 80. Here, since the first standing wave 851 and the second standing wave 852 differ in frequency, a third standing wave is synthesized from the two standing waves 851, 852 in the region between the ultrasonic oscillators 3a, 3b and the reflector 6, resulting in an irradiation pattern with such an energy intensity distribution that the energy intensity is maximized through mutual strengthening at the target site 80 and is reduced through mutual cancellation as the distance from the target site 80 increases. In addition, the wavelength λ and frequency *f* of the standing wave are equal to the wavelength λ and frequency *f* of the original ultrasound.

Fig. 29 illustrates one example of the irradiation pattern of the standing waves. In the present example, the different-frequency ultrasounds 85a, 85b are oscillated or emitted from the ultrasonic oscillators 3a, 3b, and are superposed on the ultrasounds 85a', 85b' reflected by the reflector 6 to form the standing waves 851, 852. By appropriate selection of the wavelengths (λ851, λ852) of the standing waves 851, 852, the ultrasounds are superposed on each other to strengthen the ultrasound intensity at the target site 80.

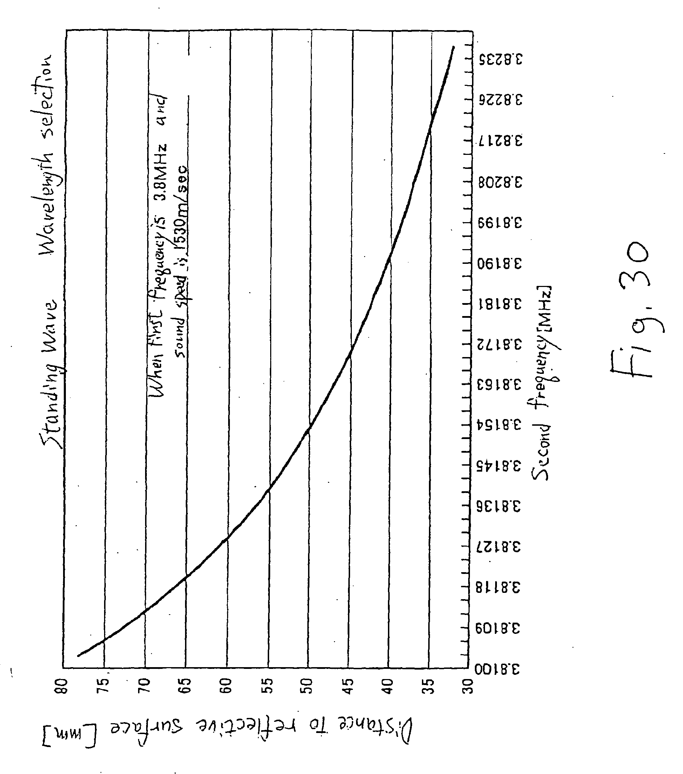

Fig. 30 is a graph showing the relationship between the distance L and the second frequency (F2) in the case where the first frequency (F1) is 3.8 MHz. In order to obtain a highest ultrasound intensity at the center of the distance L, it suffices to fulfill the condition L = 1/(2 × |1/λ851 - 1/λ852|). For example, where the distance is L = 50 mm and the first frequency (7F1) is 3.800 MHz, it suffices to set the second frequency (F2) to 3.815 MHz. The relationship between frequency and wavelength is defined as wavelength = acoustic velocity/frequency. Here, the acoustic velocity is 1530 m/seen (inside the living body).

The first wavelength λ852 of the standing wave 851 and the second wavelength λ852 of the standing wave 852 are determined by the following formulas. Namely, where the distance between the emission units 3 and the reflector 6 is L the wavelengths λ851 and λ852 are so selected as to satisfy the formulas: L = n × λ851/2 and L = m × λ852/2, where n and m are integers. Alternatively, where the wavelengths are present or known, the distance L between the main body including the emission units 3 and the reflection means 6 is controlled by the arranging apparatus 14 so that the distance L satisfies the above formulas, whereby the standing wave 851 and the standing wave 852 can be produced.

Where the target sites 80 are present at different positions in the depth direction, the maximum distribution of energy intensity of the third standing wave 85 synthesized from the first and second standing waves 851, 852 can be adjusted to the target site 80 by controlling the frequencies of the ultrasounds from the ultrasonic oscillators 3a, 3b.

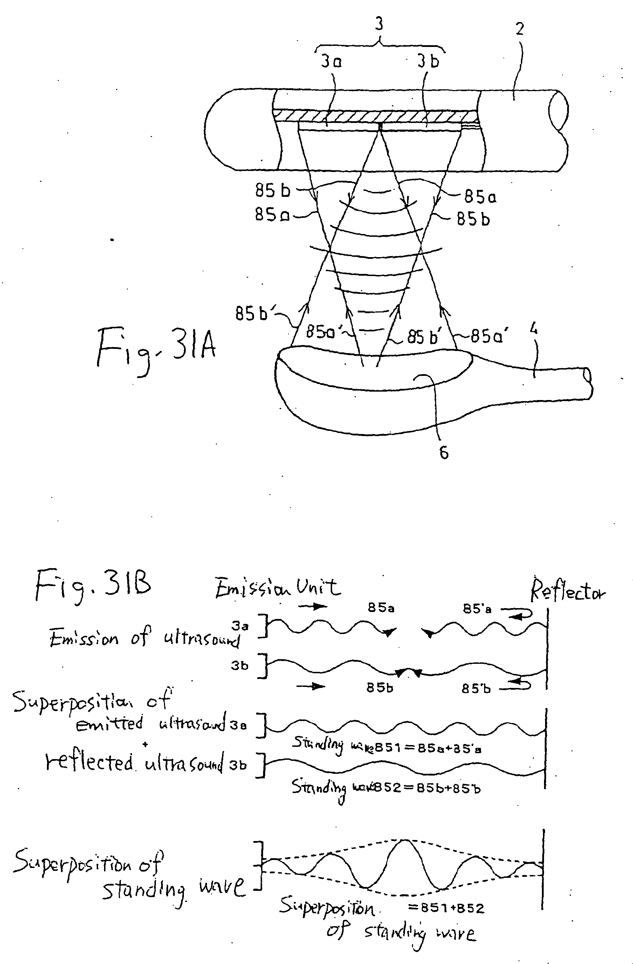

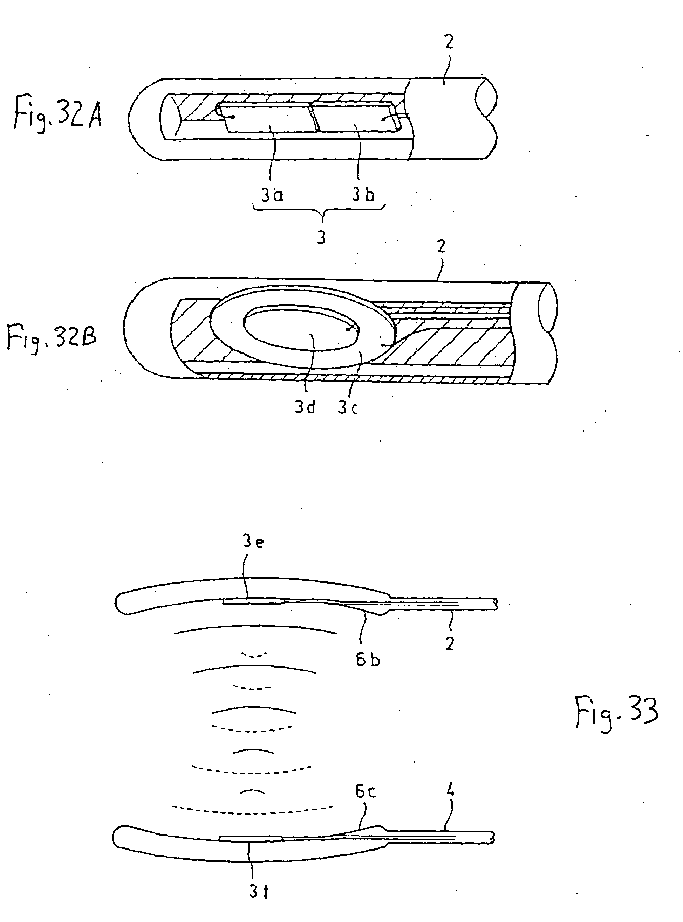

Figs. 31A and 31B schematically illustrate the irradiation condition obtained using the reflector 6 and the remission units 3a, 3b shown in Figs. 32A and 32B. The energy treatment apparatus according to the invention includes ultrasonic oscillators 3a, 3b disposed side by side in the longitudinal direction of the applicator 2 to emit ultrasounds in the same circumferential direction, and includes a reflection means 4 disposed at a position opposite to the applicator 2 which includes the ultrasonic oscillators 3a, 3b. The first ultrasound 85a radiated from the ultrasonic oscillator 3a is superposed on the ultrasound 85a' which has penetrated through the target site 80 and been reflected by the reflection means 4 to produce the first standing wave 851. Similarly, the second ultrasound 85b radiated from the ultrasonic oscillator 3b is superposed on the reflected ultrasound 85b' to produce the second standing wave 852. By setting the appropriate different frequencies and the appropriate distance 1 between the applicator 2 and the reflection means 4 as described above, it is possible to ensure that the standing wave 851 and the standing wave 852 are further superposed on each other to give a maximum intensity at the target site 80, and the energy treatment can be achieved in a concentrated manner.

Fig. 32B is a perspective view of another example of the emission unit 3 comprising two ultrasonic oscillators 3c, 3d. This version of the emission unit includes an annular ultrasonic oscillator 3c and a circular ultrasonic oscillator 3d which are concentric with one another. Ultrasounds as different-frequency standing waves can be oscillated from the two ultrasonic oscillators 3c,3d. By replacing the above-mentioned emission unit of Fig. 31A with the emission unit shown in Figs. 32A and 32B having the ultrasonic oscillators 3c, 3d, an energy treatment similar to that described above can be performed.

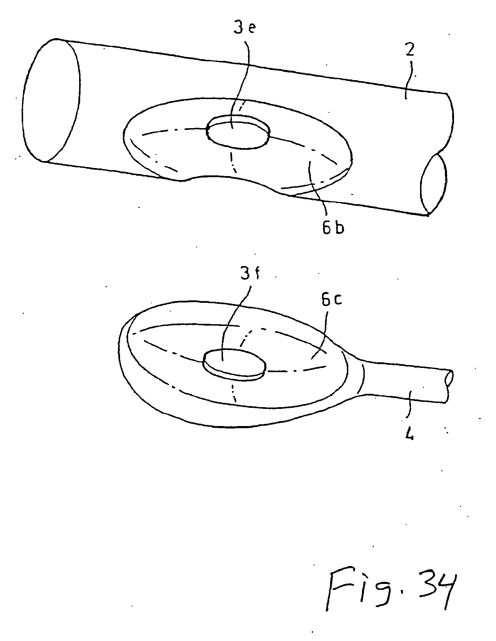

Fig. 33 is an illustration of the irradiation condition showing a further embodiment of an energy treatment apparatus according to the invention in which two assemblies each including an emission unit and reflection means arranged in an integral form as shown in Fig. 34 are arranged at positions opposite one another, with the target site located between the two assemblies. One assembly includes an ultrasonic oscillator 3e integrated with the distal end of the main body 2 for producing a first standing wave 851 and a reflector 6b of a reflection means for reflecting the ultrasound, with the reflector 6 located in surrounding relation to the ultrasonic oscillator 3e. The other assembly includes a reflector 6c of reflection means which is disposed at a position opposite to the main body and an ultrasonic oscillator 3f located at the reflector 6c (e.g., a center portion of the reflector) for producing a second standing wave 852.

The ultrasound as the first standing wave 851 produced by the ultrasonic oscillator 3e and the ultrasound as the second standing wave 852 produced by the ultrasonic oscillator 3f are superposed on each other at the target site 80 to strengthen each other at the target site 80. Energy treatment can thus be performed in a concentrated manner.

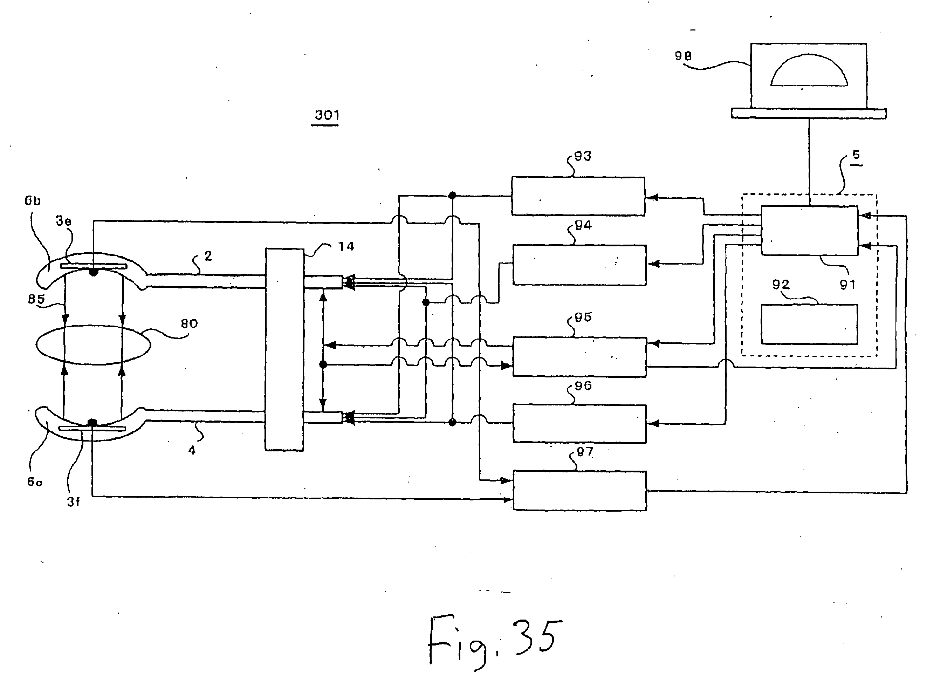

Fig. 35 shows the overall configuration of an another embodiment of the energy treatment apparatus according to the invention in which the emission unit and the reflection means of Fig. 34 are integrated with each other. Even in this version where the emission unit and the reflection means are integrated with each other, the energy treatment apparatus 301, like the embodiments described above, includes an applicator 2 which is an elongate insertion portion, an energy emission unit 3e and a reflector 6b disposed at or in the applicator 2, a reflection means 4 having an emission unit 3f and a reflector 6c opposed to the emission unit 3e and the reflector 6b, and a controller 5. The applicator 2 and an operating rod 7 of the reflection means 4 are held by an arranging apparatus 14 in an extracorporeal region. The reflector 6b reflects energy from the emission unit 3f, while the reflector 6c reflects an energy from the emission unit 3e.

The controller 5 includes a control unit 91 composed of a CPU such as a microprocessor, and a data base 92 storing programs to be executed by the control unit 91 and various kinds of data. Several control devices are connected to the control unit 91. These control devices include an emission control means 93 for setting transmission outputs and an irradiation pattern for driving the emission units 3e, 3f, for example ultrasonic oscillators, a cooling liquid control means 94 for supplying and draining a cooling liquid to and from the applicator 2 and the reflectors 6b, 6c (or the emission units 3e, 3f and the reflectors 6b, 6c), an arrangement control means 95 for setting the distance between and the directions of the applicator 2 and the reflection means 4, a reflection control means 96 for controlling, for example, the radii of curvature of the reflectors 6b, 6c, and an energy detection means 97 for detecting the intensities of the energies, for example ultrasounds, emitted from the energy emission units 3e, 3f. Further, a monitor 98, which can be of the cathode ray tube or liquid crystal type for example, is provided as a display means for displaying the results of arithmetic operations of input information, etc.

The cooling liquid control means 94 is configured for injecting and draining in a circulatory manner for example a cooling liquid so as to prevent surface layers of the living body tissues other than the target site (i.e., surface layers making contact with the applicator 2 and the reflectors 6b, 6c) from being significantly heated by the ultrasounds emitted from the ultrasonic oscillators 3e, 3f.

In connection with the various embodiments of the applicator described above, an observation means for observing living body tissues may be provided in the distal end of the applicator 2. The observation means can be, for example, an endoscope, a CCD camera or the like. As mentioned above, where an endoscope is provided, the applicator 2 is inserted into the urethra, and the urethra is then observed through use of the endoscope to observe, for example, the shape characteristics of the urethra at the prostate and to judge the forward-backward position of the target site 80 .An example of an endoscope will be described below. An endoscope uses an optical fiber functioning also for radiating an illumination beam, and is provided with an imaging lens at the distal end thereof. The endoscope can be disposed so that it can be freely inserted and retracted through a proximal end portion of the energy irradiation apparatus. Using the endoscope observation, the ultrasound emission unit 3 can be positioned as desired. In addition, through use of an endoscope provided with a guide beam function, the ultrasound irradiation position can be visually confirmed. Further, since the irradiated surface can be continuously observed during irradiation with ultrasound, the irradiation conditions can be optimized while observing the irradiated state.

The applicator 2 and the reflection means 4 are disposed to be movable so that the distance between them can be controlled by the arranging apparatus 14. Therefore, the applicator 2 and the reflection means 4 are preferably formed of materials comparatively high in hardness, and can be formed, for example, of stainless steel or the like. Possible material for constituting the applicator 2 will be described later. The applicator 2 is provided with a window (not shown) for radiating the energy emitted from the emission unit 3. The window can be used also for observation by use of the observation means which will be described later and is an endoscope in this embodiment. It is desirable to utilize a material for the window that is excellent in transmission characteristics. Examples of such materials include polyethylene terephthalate, quartz glass, acrylic resin, polystyrene, polycarbonate, polyethylene, polypropylene, polyvinylidene chloride, Teflon®, and polyester.

Also, a balloon capable of expansion and contraction for position fixation after insertion may be provided at that portion of the applicator 2 which is inserted in a living body cavity. It is preferable to use a material excellent in transmissivity to ultrasound as the material for constituting the balloon. Examples of such material include polyolefin, polyester, polyamide, latex, and cellulose. This helps ensures that energy loss or heat generation due to absorption of ultrasound by the balloon is suppressed.

As a working fluid for expanding the balloon, air and a cooling liquid can be used. The working fluid is not particularly limited as long as it enables expansion and contraction of the balloon. A cooling liquid is preferred. With cooling liquid used as the working fluid, surface layer portions of the body tissues can be cooled by the cooling liquid at the time of irradiation with ultrasound, whereby the surface layer portions can be more securely prevented from being damaged.

The temperature of the cooling liquid is not particularly limited inasmuch as the cooling liquid can cool the surface layer portions of the living body tissues, but is preferably 0°C to 37°C, more preferably about 0°C to 25°C.

Physiological saline, Ringer's solution and the like are preferably used as the working fluid. With one of these liquids used as the working fluid, it is possible to reduce the influence of leakage of the working fluid into the inside of a living body which might occur due to some cause.

Where the cooling liquid is used as the working fluid, it is preferable to circulate the cooling liquid,, It is also preferably to circulate the cooling liquid from before the irradiation with ultrasound begins until the ultrasound irradiation is finished and the treatment is over. With the cooling liquid circulated, cooling efficiency can be enhanced, and, with the cooling liquid circulated from before the start of ultrasound irradiation until the ultrasound irradiation is finished, the surface layer portions can be further cooled.

It is preferable to provide the drain portion with, for example, a pressure valve which is opened when a predetermined pressure is exceeded. This makes it possible to expand the balloon at a predetermined pressure, irrespective of the flow rate of the cooling liquid.

It is also preferable to control the temperature of the cooling liquid and the flow rate

of the cooling liquid in conjunction with the irradiation with ultrasound. This makes it possible to prevent the surface layer portions from being cooled or heated excessively.

The balloon can be provided with a temperature sensor for detecting the surface temperature of the body tissue. In this case, it is possible to detect the surface temperature of the body tissue by way of the temperature sensor, and to utilize the thus obtained information (detected value) to control cooling. This helps contribute to an efficient, necessary and sufficient cooling.

The balloon may be formed to surround the whole circumference of the housing, exclusive of the ultrasound emission area. In this case, expanding the balloon presses the ultrasound emission area of the main body against the body cavity wall so that the distance between the target of irradiation and the emission unit is made stable, leading to relatively good stability at the time of irradiation.

A hard pipe made of, for example, a metal such as stainless steel is preferably used as the material for constituting the applicator 2. Examples of the material for constituting the main body of the applicator include polyolefins such as polyethylene, polypropylene, etc.; ethylene-vinyl acetate copolymer (EVA); polyvinyl chloride; polyesters such as polyethylene terephthalate, polybutylene terephthalate, etc.; polyamide; polyurethane; polystyrene; polycarbonate; fluororesin; polymer alloys containing one of these materials, and combinations of two or more of these materials.

In addition, the surface of the main body may be provided with a lubricating coating formed of a hydrophilic polymer material, silicone, fluororesin or the like. This reduces friction on the main body surface, and allows for a smoother insertion of the applicator 2 into a body cavity.

Preferable examples of the hydrophilic polymer material to be used for the lubricating coating include carboxymethyl cellulose, polysaccharide, polyvinyl alcohol, polyethylene oxide, poly(sodium acrylate), methyl vinyl ether-maleic anhydride copolymer, and watersoluble polyamide. Among these, methyl vinyl ether-maleic anhydride copolymer is particularly preferred.

In the case of using the energy treatment apparatus with the main body coated with the hydrophilic polymer material, the surface layer of the energy treatment apparatus is immersed, for example, in physiological saline or the like. This wets the surface layer, giving lubricity to the surface of the energy treatment apparatus. Since the energy treatment apparatus has the surface layer containing the hydrophilic polymer material, friction between the living body tissues and the energy treatment apparatus is reduced, whereby the burden on the patient is alleviated, and safety is enhanced. For example, it is possible to smoothly perform the insertion of the energy treatment apparatus into a body cavity, the pulling of the energy treatment apparatus out of the body cavity, and the movement and rotation of the energy treatment apparatus in the body cavity.

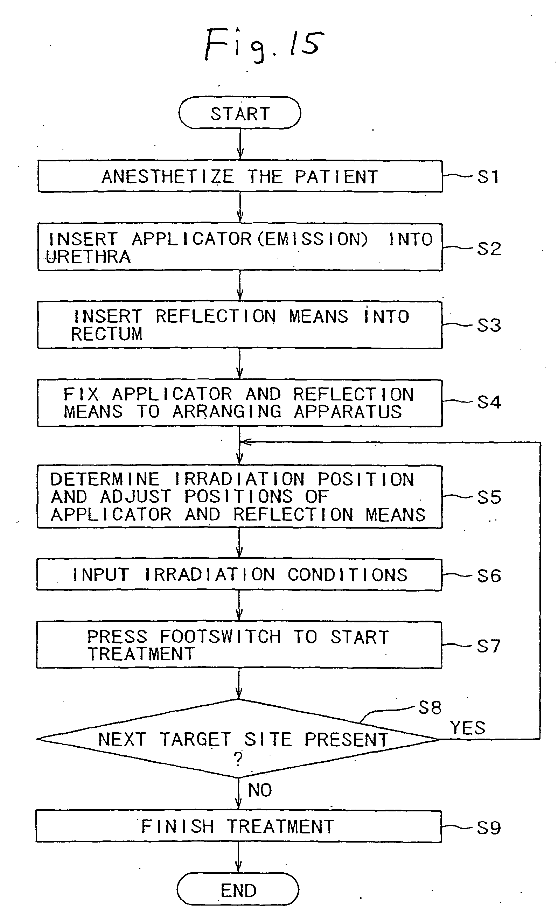

Fig. 15 is a flowchart showing a treatment using the energy treatment apparatus 1 illustrated in Fig. 14. First, the patient is anesthetized in step S 1. The anesthesia may be general anesthesia or local anesthesia. In step S2, the applicator 2 is inserted into the urethra. In step S3, the reflection means 4 is inserted into the rectum. In step 4, the applicator 2 and the reflection means 4 are fixed to the arranging apparatus 14. In step S5, an irradiation position of a target site (a site to be treated) is determined, and the positions of the applicator 2 and the reflection means 4 are adjusted. In step S6, ultrasound irradiation conditions are inputted to the controller 5. In step S7, a footswitch 114 is pressed to start the treatment. In step S8 it is determined whether there is another target site requiring treatment. If it is judged in step S8 that there are no other target sites for treatment, the operation proceeds to step S9 in which the treatment is finished. On the other hand, if it is judged in step S8 that another target site exists, the process returns to step S5, and steps S5 through S8 are repeated until there is no target site left to be treated.

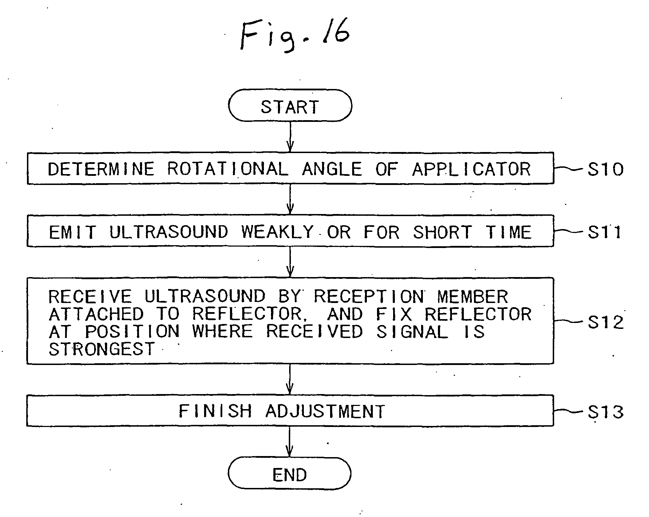

Fig. 16 is a flowchart of a process for adjusting the position of the target site. In step S10, a rotational angle about the axis of the applicator 2 is determined, so that the irradiation direction of the emission unit (ultrasonic oscillator) 3 is determined. In step S11, the ultrasound is radiated weakly or for a short time. In step S 12, the ultrasound is received by a reception member attached to the reflector 6, in this embodiment by the ultrasound sensor 35, and the reflector 6 is fixed at the position where the received ultrasound is the strongest. In step S13, the adjustment is finished.

With the energy treatment apparatus 1 as above-described, the target site 80 is irradiated with both the ultrasound from the ultrasonic oscillator 3 and the reflected ultrasound from the reflector 6 in a synchronous manner so that both ultrasounds (the reflected ultrasound from one emission and the ultrasound from the next emission) are superposed on each other at the target site 80, thus enhancing the efficiency of accumulation of ultrasound into or at the target site 80 together with the ultrasound utilization efficiency, and to heat the target site to a relatively optimum temperature for high-temperature treatment.

The use of the depth-reaching ultrasound as the irradiation energy makes it possible to apply a high-temperature treatment to a site located at a depth.

Also with the ultrasound emission unit 3 repeatedly reciprocated in the axial direction by the electric drive means 157, only the target site 80 can be irradiated in a concentrated manner with the ultrasound, without giving significant damage to the normal living tissues in the surroundings. In addition, the ultrasound accumulation efficiency can be increased so that a heating temperature appropriate for the target site can be attained even where the ultrasound emission output is reduced.

Where the drive means 157, for example, constituting the moving means for moving the ultrasound emission unit 3 is provided with an adjusting means for adjusting the moving amount of the reciprocating movement of the emission unit 3, an ultrasound treatment from a relatively optimum position can be achieved at the time of irradiation with ultrasound.

Since the applicator 2 and the reflection means 4 can be moved, by the arranging apparatus 14, in the longitudinal direction of the applicator 2 and/or in a direction orthogonal to the longitudinal direction according to the target site 80, the ultrasound radiated from the ultrasonic oscillator 3 can be set to a relatively optimum position for the target site 80. Also, it is possible to perform positioning while observing.

The description set forth above describes embodiments of the energy treatment apparatus in which two standing waves are radiated from two ultrasound emission units to strengthen each other on an energy basis. It is to be understood that three or more standing waves may be adopted, whereby the accumulation of energies at the target site can be enhanced further. The wavelengths of the respective standing waves are set so that the distance L between the emission unit and the reflector is an integer times of one-half of the wavelength of each of the standing waves. Also, with the energy treatment apparatus 1 according to embodiment as described above, the target site 80 is irradiated with both the respective different-frequency ultrasounds from the plurality of ultrasonic oscillators and the respective reflected ultrasounds from the reflectors so that both the emitted ultrasounds and the reflected ultrasounds are superposed on each other at the target site 80 to produce the plurality of different-frequency standing waves from which a standing wave is synthesized. It is thus possible to enhance the efficiency of accumulation of ultrasounds into the target site 80 together with the ultrasound utilization efficiency, and to heat the target site to a relatively optimum temperature for high-temperature treatment.

The controller 5 controls the energy emission unit 3 and the reflection means 4, so that the treatment can be performed more efficiently and speedily. Since the arranging apparatus 14 can arrange the energy emission unit 3 and the reflection means 4, it is possible to stabilize the positions of the energy emission unit 3 and the reflection means 4 relative to the target site, and to irradiate the target site accurately at the time of irradiation with the energy.

By providing the cooling means for the applicator 2, it is possible to cool the surface layer portions of the living body tissues with the cooling liquid, and to more readily prevent the surface layer portions from being damaged.

Using the acoustic lens 23 for converging, collimating, or diverging the ultrasound provided at the ultrasound emission surface of the ultrasonic oscillator 3, it is possible to irradiate the target site with the ultrasound in a wider region or a narrower region or in the manner of concentrating the ultrasound, depending on the state of the target site.

The cooling means provided for the reflection means 4 makes it possible to cool the surface layer portions of living body tissues with the cooling liquid, and to prevent more securely the surface layer portions from being damaged.

Providing the reflection means 4 with the fixed reflective surface or the variable reflective surface, it is possible to use an optimum reflective surface according to the treatment range of the target site to thus achieve a more efficient treatment.

Since the arranging apparatus 14 can be provided with a changing function for changing the radiation direction of ultrasound from the ultrasonic oscillator 3, the ultrasonic oscillator 3 can be changed to an appropriate position relative to the target site, thus shortening the setting time.

With the curvature of the reflective surface of the reflection means 6 made variable, the irradiation range can be varied according to the position in the depth direction of the target site and the area of the target site.

By providing the reflector 6 with the ultrasound sensor 35, it is possible to detect the output intensity of the ultrasound, to control the output of the ultrasound emitted from the ultrasonic oscillator 3, and to reconsider the settings of conditions.

In the energy treatment apparatus disclosed here, in arrangements in which the reflector 6 is provided with an air bleeding hole 36 and an ultrasound jelly injection hole 37, injecting an ultrasound jelly into the reflector 6 makes it possible to bring the reflector 6 into close contact with a body cavity wall. This allows the ultrasound to be more accurately reflected toward the target site 80 without significant irregular reflection of the ultrasound.

Providing the arranging apparatus 14 for holding the main body and the reflector 6, the main body and the reflector 6 can be fixed at relatively optimum positions, and so energy irradiation can be performed relatively stably.

By making it possible to produce an irradiation pattern for concentrating the energy into or at the target site designated by the controller 5, relatively efficient treatment of the target site is possible.

As described above, the controller 5 can control the cooling means for the main body and the cooling means for the reflection means. This allows the surface layer portions of living body tissues to generally always be cooled with the cooling liquid. This thus more reliably inhibits or prevents the surface layer portions from being damaged and so a relatively speedy and efficient treatment can be achieved.

Also, since the controller 5 controls the reflection means 4, by varying the curvature of the fixed reflective surface or variable reflective surface, it is possible to adjust the irradiation range to an irradiation range according to the position in the depth direction of the target site and the area of the target site, and to achieve a relatively speedy and efficient treatment.

When an endoscope is arranged in the applicator 2, it is possible to observe the state of the target site before and after a treatment, and generally optimize the irradiation conditions while observing the state.

When the applicator 2, and possibly also the reflection means 4, is provided with the balloon capable of expansion and contraction, the applicator 2 for example can be pressed against a body cavity wall in the vicinity of the target site 80. The distance between the target site 80 and the ultrasonic oscillator 3 can thus be made more stable, and the irradiation of the target site 80 with the ultrasound can be better stabilized.

Applying a surface layer including a hydrophilic polymer material on the surface of the applicator 2, and possibly also the reflector means 4, makes it possible to reduce the friction, for example the friction on the surface of the applicator 2. Thus, in the case of the applicator, it is possible to more smoothly insert the applicator 2 into a body cavity, pull the applicator 2 out of the body cavity, and move and rotate the applicator 2 inside the body cavity.

As described above, the main body, the reflection means, and the balloon can be provided respectively with cooling means. However, the cooling means for the main body, the reflection means and the balloon can be provided in any combination desired, according to the cooling conditions.

The embodiments described above have been described in the context that the energy radiated toward the living body tissue is ultrasound. However, the present invention is not limited to these examples, and the invention is applicable also to irradiation with other energies, for example laser beams, electromagnetic waves having directivity, and others. In such situations, if necessary, the applicator can be provided in its area corresponding to the emission unit with a window for ensuring that irradiation with the energy is not hindered. While the examples described above have been discussed in the context that the living body tissue which is the object of high-temperature treatment is the prostate, the present invention is not limited in this regard. The object of treatment includes all living body tissues that can be subjected to high-temperature treatment by irradiating them with an energy from the inside of a living body, i.e., from a blood vessel, a digestive tract (esophagus, intestines, etc.), abdominal cavity, or the like, or from the body surface.

Since the embodiments of the energy treatment apparatus described here include the energy emission unit(s) and the reflection means, the energy utilization efficiency is enhanced. It is thus possible to enhance the accumulation of energy into or at the target site and, hence, the intensity of the energy, while also generally suppressing the emission output. Therefore, it is possible to beat the target site to a temperature relatively optimum for high-temperature treatment, and to perform an efficient high-temperature treatment while using a relatively low output. In addition, the energy treatment apparatus can be configured, as described by way of example above, so that the energy emitted from the emission unit(s) is collected into the target site by way of different routes, thus increasing the efficiency of accumulation of energy into the target site. Therefore, it is possible to heat the target site to a temperature relatively optimum for high-temperature treatment, and to perform thermotherapy in a relatively short time.

Since the energy treatment apparatus includes the energy emission unit(s) and the reflection means, the energy utilization efficiency is enhanced, and it is possible to enhance the accumulation of energy into the target site and, hence, the intensity of the energy, while suppressing the emission output. Also, the energy emission unit(s) of the illustrative examples as capable of being moved and so the target site can be irradiated with the energy coming through different paths. Since the target site is irradiated with energy while moving the energy emission unit(s), the efficiency of accumulation of the energy into the target site can be further enhanced, without exerting any significant influence on the living body tissues other than the target site.

Where the energy treatment apparatus utilizes an elongate main body having the energy emission unit(s) and a moving means by which the position of the energy emission unit(s) disposed in the main body is moved in the axial direction of the main body, the energy can be efficiently accumulated into the target site while suppressing the heating of body tissues other than the target site. Also, when the energy treatment apparatus includes the moving means by which the position of the energy emission unit disposed in the main body is repeatedly reciprocated in the axial direction of the main body, only the target site is irradiated in a concentrated manner with the energy, without significantly damaging the normal living tissues surrounding the target site. It is thus possible to enhance the energy accumulation efficiency so as to attain an appropriate heating temperature for the target site, even where the energy emission output is set relatively low.

By providing a moving means for the energy emission unit(s) to generally ensure that the energy emitted from each moved position of the energy emission unit(s) is concentrated into or at the target site, the efficiency of accumulation of the energy into the target site can be enhanced. Also, where the moving means is provided with a movement control means by which the movement amount and moving speed of the reciprocating movement of the energy emission unit(s) can be regulated, it is possible to achieve an energy treatment by irradiation with the energy from a relatively optimum position.

Also, the emission control means provided for controlling the emission of the energy emitted from the energy emission unit(s) allows the emission pattern of the energy to be controlled. The energy can thus be efficiently accumulated into or at the target site.

Embodiments of the energy treatment apparatus according to the present invention include a plurality of energy emission units for emitting energies of different-frequency standing waves and reflection means, with the target site being irradiated with energy obtained by superposing the different-frequency standing waves on each other. The energy utilization efficiency is thus enhanced, and it is possible to enhance the accumulation of energy into the target site and hence the intensity of the energy, while also suppressing the emission output. It.is thus possible to heat the target site to a temperature for high-temperature treatment and to perform an efficient high-temperature treatment while using a relatively low output.

It is also possible to ensure that the energy obtained through superposition of the standing waves by controlling the energy emission patterns and the distance between the emission units and the reflection means provides an energy intensity distribution such that the energy intensity is relatively high at the target site. At the same time, energies can be efficiently accumulated at or into the target site.

By superposing the energies which partly penetrate through the target site and are reflected by the reflection means on the energies from the energy emission units to produce different-frequency standing waves and by irradiating the target site with the different-frequency standing waves in a further superposed manner, the energy accumulation is enhanced and the target site can be heated to a relatively optimum temperature.

Additionally, irradiating the target site with the energies of different-frequency standing waves from the plurality of energy emission units makes it possible to superpose the energies of the standing waves on each other in generally optimum conditions according to each of target sites which may differ in depth, thus enhancing the accumulation of energies heating the target site to an optimum temperature.

In embodiments of the energy treatment apparatus described above, a plurality of energy emission units are arranged on one side, and reflection means is arranged at a position opposite to the plurality of energy emission units with the target site therebetween. Therefore, energy utilization efficiency is enhanced. In addition, it is possible to enhance the accumulation of energies into the target site and, hence, the energy intensity, while suppressing the emission outputs. The target site can thus be heated to a temperature sufficient for high-temperature treatment, and relatively efficient high-temperature treatment can be achieved using relatively low outputs.

Embodiments of the energy treatment apparatus described above utilize energy emission units which each integrally include reflection means and which are arranged at opposite positions with the target site therebetween. This also allows enhancement of energy utilization efficiency, and makes it possible to enhance the accumulation of energies into or at the target site and hence the energy intensity, while suppressing the emission outputs. The target site can thus be heated to a temperature for high-temperature treatment, and efficient high-temperature treatment can be performed while using relatively low outputs.

Irradiating the target site with the energy from the energy emission unit(s) makes it possible to ensure that a part of the energy penetrates through the target site and is reflected by the reflection means and that the reflected energy and the next energy are superposed on each other at the target site, to enhance the accumulation of energy, and to heat the target site to an optimum temperature. Also, by irradiating the target site with both the energy partly penetrating through the target site and reflected by the reflection means and the next energy pulse from the energy emission unit(s) in such a manner that both energies are superposed on each other at the target site, the energy accumulation is enhanced and the target site can be heated to a desired or relatively optimum temperature.

The arranging apparatus can arrange both a main body including the energy emission unit(s) and the reflection means. Thus, the positions of the main body and the reflection means relative to the target site can be stabilized, and the target site can be accurately irradiated with the energy at the time of irradiation with the energy.

Providing the arranging apparatus with a holding unit for the main body including the energy emission unit(s), a holding unit for the reflection means, and a mechanism for adjusting the relative positions of the energy emission unit(s) and the reflection means makes it possible to fix the energy emission unit(s) and the reflection means at desired or relatively optimum positions to facilitate generally stable irradiation with the energy. Also, with an arrangement control means provided for controlling the arranging apparatus, irradiation with energy can be efficiently performed in a relatively optimum layout.

Since the arrangement control means performs a control allowing the emitted energy from the energy emission unit(s) and the reflected energy from the reflection means to be superposed on each other at the target site, it is possible to perform treatment more efficiently and speedily. Also, since the arranging apparatus is capable of changing the direction of the energy emission unit(s), the position of the emission unit(s) can be changed to an appropriate direction relative to the target site, and the setting time can be shortened.

When the emission of the energy from the energy emission unit(s) is controlled by the emission control means in conjunction with the energy reflected by the reflection means, it is possible to superpose the emitted energy and the reflected energy at the target site, and thereby efficiently accumulate the energy. Also, because the emission control means controls the irradiation pattern for concentrating the emitted energy into the target site, the irradiation output, and the irradiation time, it is possible to treat the target site optimally, efficiently, and speedily. Further, because the emission control means controls the irradiation pattern to intermittently irradiate the target site with the emitted energy from the emission unit(s), it is possible to perform a speedy and efficient treatment. Further, because the emission control means controls the irradiation pattern so that the energy intermittently emitted from the energy emission unit(s) and the reflected energy reflected by the reflection means will reach the target site simultaneously, it is possible to achieve a speedy and efficient treatment. With the reflection means provided with a fixed reflective surface or a variable reflective surface, an optimum reflective surface can be used and a more efficient treatment can be performed, according to the range of treatment of the target site. Also, with the reflection means formed so that the radius of curvature of its reflective surface is variable, the irradiation range can be varied according to the position in the depth direction of the target site and the area of the target site. By providing the reflection control means for controlling the reflection means, it is possible to perform the treatment more efficiently and speedily. Also, providing the reflection means with an energy detection means for detecting the reaching efficiency of energy, the emitted energy can be detected, making it possible to control the output of the emitted energy and to possibly reconsider the settings of the irradiation conditions. Where the reflector is provided with an air bleed hole, it is possible to bleed air and to bring the reflector into close contact with a body cavity wall. The energy radiated from the emission unit(s) can thus be reflected accurately toward the target site, without significant irregular reflection.

With a cooling means provided at the energy emission unit(s) or at a main body including the energy emission unit(s), surface layer portions of living body tissues can be cooled with a cooling liquid, and the surface layer portions can be inhibited or prevented from being damaged. Also, with a cooling means provided for the reflection means, the temperature of living body tissues in the vicinity of the reflection means can be prevented from being raised, and the treatment of the target site can be performed efficiently and stably. In addition, by providing cooling control means for controlling the cooling means, surface layer portions of body tissues can be cooled with cooling liquid to prevent damage to the surface layer portions and allow a relatively speedy and efficient treatment to be performed. It is also possible to reduce the possibility that the temperature of the living body tissues in the periphery of the reflection means will be excessively raised, thus allowing the treatment of the target site to be relatively efficiently and stably performed.

The controller provided for controlling the movement control means allows a relatively speedy and efficient treatment to be realized through automation. Also, with a controller provided for controlling the arrangement control means or controlling both the arrangement control means and the movement control means, it is possible to relatively optimally set the positions of the emission unit and the reflection means relative to the target site, and to perform an efficient and speedy treatment through automation. Providing a controller for controlling the arrangement control means or for controlling both the arrangement control means and the emission control means makes it possible to achieve an efficient and speedy treatment through automation of the relevant function(s). Additionally, with a controller provided for controlling the reflection control means or controlling at least two of the reflection control means, the arrangement control means, and the emission control means, an efficient and speedy treatment can be performed through automation of the relevant function(s). Also, by providing a controller for controlling the cooling control means or controlling at least two of the cooling control means, the arrangement control means, the emission control means, and the reflection control means, an efficient and speedy treatment can be achieved through automation of the relevant function(s).

By providing a controller for controlling the emission control means or controlling at least two of the emission control means, the arrangement control means, and the movement control means, a relatively efficient and speedy treatment can also be achieved through automation of the relevant function(s). A similar result can be achieved with a controller provided for controlling the reflection control means or controlling at least two of the reflection control means, the emission control means, the arrangement control means, and the movement control means. A relatively efficient and speedy treatment can also be performed through automation of the relevant function(s) by providing a controller for controlling the reflection control means or controlling at least two of the reflection control means, the emission control means, the arrangement control means, and the movement control means, Additionally, with a controller provided for controlling the cooling control means or controlling at least two of the cooling control means, the reflection control means, the emission control means, the arrangement control means, and the movement control means, a relatively efficient and speedy treatment can be achieved through automation of the relevant function(s).

Where a main body including the energy emission unit(s) is provided with means for converging, collimating, or diverging the spreading angle of the emitted energy, it is possible to radiate the energy to a wider area or a narrower area and to irradiate the target site in a concentrated manner with the ultrasound, depending on the state of the target site. Where a main body including the energy emission unit(s)is provided with observation means, the state of the target site before and after the treatment can be observed, and the irradiation conditions can be relatively optimized while observing the state.

Where the energy radiated from the emission unit(s) is ultrasound, a site located in the depth can be selectively irradiated with ultrasound.

The principles, preferred embodiments and modes of operation of the present invention have been described in the foregoing specification. However, the invention which is intended to be protected is not to be construed as limited to the particular embodiments described. Further, the embodiments described herein are to be regarded as illustrative rather than restrictive. Variations and changes may be made by others, and equivalents employed. Accordingly, it is expressly intended that all such variations, changes and equivalents which fall within the scope of the invention be embraced by the appended claims.

## Claims

1. An energy treatment apparatus (201, 301) for irradiating living body tissue with energies, comprising:
energy emission means (3) for emitting energy toward a target site (80) of living body tissue,
the energy emission means (3) being adapted to emit energy having first and second different frequencies, **characterized by**
reflection means (4) positionable relative to the energy emission means (3) toward said target site (80) of living body tissue,
wherein the energy emission means (3) and the reflection means (4) are configured according to one of the following alternatives (i), (ii) or (iii):
(i) the energy emission means (3) comprises a first respectively a second ultrasonic oscillator (3a, 3b) disposed side by side within one applicator (2), the reflection means (4) is configured to be disposed at a position opposite to the applicator (2) such that a first respectively a second standing wave (851, 852) is produced by superposition of first respectively second ultrasound (85a, 85a') radiated from the first respectively second ultrasonic oscillator (3a, 3b) with respective ultrasound reflected from the reflection means (4);
(ii) the energy emission means (3) comprises a first annular respectively a second circular ultrasonic oscillator (3c, 3d) disposed concentric with one another within one applicator (2), the reflection means (4) is configured to be disposed at a position opposite to the applicator (2) such that a first respectively a second standing wave (851, 852) is produced by superposition of first respectively second ultrasound (85a, 85a') radiated from the first respectively second ultrasonic oscillator (3c, 3d) with respective ultrasound reflected from the reflection means (4);
(iii) the energy emission means and the reflection means are embodied in two assemblies each including an emission means and reflection means arranged in integral form, the two assemblies being able to be arranged at positions opposite to one another, the first respectively second assembly each comprises an ultrasonic oscillator (3e, 3f) and a reflector (6b, 6c) located in surrounding relation to the ultrasonic oscillator (3e, 3f), whereby a first standing wave (851) is produced by the ultrasonic oscillator (3e) in the first assembly and the reflector (6c) in the second assembly and a second standing wave (852) is produced by the ultrasonic oscillator (3f) in the second assembly and the reflector (6b) in the first assembly;
wherein in each of the alternatives (i), (ii) and (iii) the first and second standing wave (851, 852) are superposed on each other to give a maximum intensity at the target site (80); and by the first standing wave (851) and the second standing wave (852) having different frequencies from one another.

2. The energy treatment apparatus as set forth in claim 1, wherein the first and second wavelengths (λ851, λ852) corresponding to the first and second frequency (f1, f2) are each defined as wavelength = acoustic velocity/frequency, and, in order to obtain a highest ultrasound intensity at the center of the distance (L) between the emission unit (3) and the reflector (6) are set so as fulfil the condition L = 1/(2 × (| 1/λ851 - 1/λ852 |)).

3. The energy treatment apparatus as set forth in claim 1, wherein where the distance between the emission unit (3) and the reflector (6) is L, the wavelengths (λ851, λ852) of the respective radiated energies are so set that an integer times one half of the wavelength of each of the standing waves (851, 852) are equal to the distance (L) between the emission unit (3) and the reflector (6), i.e. L = n × λ851 / 2 and L = m × λ852 / 2, where n and m are integers.

4. The energy treatment apparatus as set forth in claim 1, wherein where the wavelengths (λ851, λ852) of the respective radiated energies are present or known, the distance (L) between the emission unit (3) and the reflector (6) is controlled so that the wavelengths (λ1, λ2) satisfy the formulas L = n × λ851 / 2 and L = m × λ852 / 2, where n and m are integers.

5. The energy treatment apparatus as set forth in claim 1, wherein the energy emission unit (3) comprises several emission units for emitting different-frequency energies.

## Patentansprüche

1. Eine Energiebehandlungsvorrichtung (201, 301) zum Bestrahlen von lebendem Körpergewebe mit Energien, umfassend:
Energieemissionsmittel (3) zum Emittieren von Energie in Richtung auf eine Zielstelle (80) des lebenden Körpergewebes,
wobei die Energieemissionsmittel (3) dazu ausgebildet sind, Energie mit ersten und zweiten verschiedenen Frequenzen zu emittieren, **gekennzeichnet durch** Reflexionsmittel (4), die relativ zu den Energieemissionsmitteln (3) in Richtung auf die Zielstelle (80) des lebenden Körpergewebes positionierbar sind,
wobei die Energieemissionsmittel (3) und die Reflexionsmittel (4) gemäß einer der folgenden Alternativen (i), (ii) oder (iii) gestaltet sind:
(i) die Energieemissionsmittel (3) umfassen einen ersten bzw. einen zweiten Ultraschalloszillator (3a, 3b), die innerhalb eines Applikators (2) Seite an Seite angeordnet sind, wobei die Reflexionsmittel (4) so gestaltet sind, dass sie an einer Position gegenüberliegend zu dem Applikator (2) so angeordnet sind, dass eine erste bzw. eine zweite stehende Welle (851, 852) erzeugt wird **durch** Überlagerung von von dem ersten bzw. zweiten Ultraschalloszillator (3a, 3b) abgestrahltem, erstem bzw. zweitem Ultraschall (85a, 85a') mit entsprechendem, von den Reflexionsmitteln (4) reflektiertem Ultraschall;
(ii) die Energieemissionsmittel (3) umfassen einen ersten ringförmigen bzw. einen zweiten kreisförmigen Ultraschalloszillator (3c, 3d), die konzentrisch zueinander innerhalb eines Applikators (2) angeordnet sind, wobei die Reflexionsmittel (4) so gestaltet sind, dass sie an einer Position gegenüberliegend dem Applikator (2) angeordnet werden, so dass eine erste bzw. eine zweite stehende Welle (851, 852) erzeugt wird **durch** Überlagerung von von dem ersten bzw. zweiten Ultraschalloszillator (3c, 3d) abgestrahltem erstem bzw. zweitem Ultraschall (85a, 85a') mit entsprechendem, von den Reflexionsmitteln (4) reflektiertem Ultraschall;
(iii) die Energieemissionsmittel und die Reflexionsmittel sind in zwei Baueinheiten ausgebildet, die jeweils ein Emissionsmittel und Reflexionsmittel umfassen, die in einer integralen Art angeordnet sind,
wobei die zwei Baueinheiten in der Lage sind, an einander gegenüberliegenden Positionen angeordnet zu werden, wobei die erste bzw. zweite Baueinheit jeweils einen Ultraschalloszillator (3e, 3f) und einen Reflektor (6b, 6c) umfasst, der in einer umgebenden Beziehung zu dem Ultraschalloszillator (3e, 3f) angeordnet ist, umfasst, wobei eine erste stehende Welle (851) von dem Ultraschalloszillator (3e) in der ersten Baugruppe und dem Reflektor (6c) in der zweiten Baugruppe erzeugt wird, und eine zweite stehende Welle (852) von dem Ultraschalloszillator (3f) in der zweiten Baueinheit und dem Reflektor (6b) in der ersten Baueinheit erzeugt wird;
wobei in einer jeweiligen der Alternativen (i), (ii) und (iii) die erste und zweite stehende Welle (851, 852) einander so überlagert sind, dass an der Zielstelle (80) eine maximale Intensität erzeugt wird; und **dadurch** dass die erste stehende Welle (851) und die zweite stehende Welle (852) voneinander verschiedene Frequenzen aufweisen.

2. Die Energiebehandlungsvorrichtung nach Anspruch 1,
wobei die erste und zweite Wellenlänge (λ851, λ852), die der ersten und der zweiten Frequenz (f1, f2) entsprechen, jeweils als Wellenlänge = akustische Geschwindigkeit / Frequenz definiert sind, und wobei, um die höchste Ultraschallintensität an dem Mittelpunkt des Abstands (L) zwischen der Emissionseinheit (3) und dem Reflektor (6) zu erzielen, so eingestellt sind, dass die Bedingung L = 1/(2 × (|1/λ851 - 1/λ852|)) erfüllt ist.

3. Die Energiebehandlungsvorrichtung nach Anspruch 1,
wobei, wenn der Abstand zwischen der Emissionseinheit (3) und dem Reflektor (6) L ist, die Wellenlängen (λ851, λ852) der entsprechenden abgestrahlten Energien so eingestellt sind, dass ein ganzzahliges Vielfaches einer halben der Wellenlänge einer jeweiligen der stehenden Wellen (851, 852) gleich dem Abstand (L) zwischen der Emissionseinheit (3) und dem Reflektor (6) ist, d.h. L = n × λ851/2 und L = m × λ852/2, wobei n und m ganze Zahlen sind.

4. Die Energiebehandlungsvorrichtung nach Anspruch 1,
wobei, wenn die Wellenlängen (λ851, λ852) der entsprechenden abgestrahlten Energien vorhanden oder bekannt sind, der Abstand (L) zwischen der Emissionseinheit (3) und dem Reflektor (6) so gesteuert wird, dass die Wellenlängen (λ1, λ2) die Gleichungen L = n × λ851 / 2 und L = m × λ852 / 2 erfüllen, wobei n und m ganze Zahlen sind.

5. Die Energiebehandlungsvorrichtung nach Anspruch 1,
wobei die Energieemissionseinheit (3) mehrere Emissionseinheiten zum Emittieren von Energien mit verschiedenen Frequenzen umfasst.

## Revendications

1. Appareil de traitement par énergie (201, 301) pour irradier des tissus de corps vivant avec des énergies, comprenant :
un moyen d'émission d'énergie (3) pour émettre de l'énergie vers un site cible (80) de tissus de corps vivant,
le moyen d'émission d'énergie (3) étant adapté pour émettre de l'énergie ayant des première et deuxième fréquences différentes,
**caractérisé par** :
un moyen de réflexion (4) pouvant être positionné par rapport au moyen d'émission d'énergie (3) vers ledit site cible (80) de tissus de corps vivant,
dans lequel le moyen d'émission d'énergie (3) et le moyen de réflexion (4) sont configurés selon l'une des variantes suivantes (i), (ii) ou (iii) :
(i) le moyen d'émission d'énergie (3) comprend un premier, respectivement un deuxième, oscillateur à ultrasons (3a, 3b) disposés côte à côte dans un applicateur (2), le moyen de réflexion (4) est configuré pour être disposé en une position opposée à l'applicateur (2) de sorte qu'une première, respectivement une deuxième, onde stationnaire (851, 852) est produite par superposition de premiers, respectivement deuxièmes, ultrasons (85a, 85a') rayonnant depuis le premier, respectivement le deuxième, oscillateur à ultrasons (3a, 3b) avec des ultrasons respectifs réfléchis par le moyen de réflexion (4) ;
(ii) le moyen d'émission d'énergie (3) comprend un premier oscillateur annulaire, respectivement un deuxième oscillateur circulaire, à ultrasons (3c, 3d) concentriques entre eux dans un applicateur (2), le moyen de réflexion (4) est configuré pour être disposé en une position opposée à l'applicateur (2) de sorte qu'une première, respectivement une deuxième, onde stationnaire (851, 852) est produite par superposition de premiers, respectivement deuxièmes, ultrasons (85a, 85a') rayonnant depuis le premier, respectivement le deuxième, oscillateur à ultrasons (3a, 3b) avec des ultrasons respectifs réfléchis par le moyen de réflexion (4) ;
(iii) le moyen d'émission d'énergie et le moyen de réflexion sont incorporés dans deux ensembles comprenant chacun un moyen d'émission et un moyen de réflexion agencés d'un seul tenant, les deux ensembles étant aptes à être agencés en des positions opposées l'une à l'autre, le premier, respectivement le deuxième, ensemble comprenant chacun un oscillateur à ultrasons (3e, 3f) et un réflecteur (6b, 6c) positionné en relation environnante par rapport à l'oscillateur à ultrasons (3e, 3f), moyennant quoi une première onde stationnaire (851) est produite par l'oscillateur à ultrasons (3e) dans le premier ensemble et le réflecteur (6c) dans le deuxième ensemble et une deuxième onde stationnaire (852) est produite par l'oscillateur à ultrasons (3f) dans le deuxième ensemble et le réflecteur (6b) dans le premier ensemble ;
dans lequel, dans chacune des variantes (i), (ii) et (iii), les première et deuxième ondes stationnaires (851, 852) sont superposées l'une sur l'autre pour donner une intensité maximale au niveau du site cible (80) ;
et par le fait que la première onde stationnaire (851) et la deuxième onde stationnaire (852) ont des fréquences différentes l'une de l'autre.

2. Appareil de traitement par énergie selon la revendication 1,
dans lequel les première et deuxième longueurs d'onde (λ851, λ852) correspondant aux première et deuxième fréquences (f1, f2) sont définies selon la relation longueur d'onde = vitesse acoustique/fréquence, et, afin d'obtenir une intensité ultrasonore la plus élevée au centre de la distance (L) entre l'unité d'émission (3) et le réflecteur (6), sont choisies de manière à satisfaire l'égalité : L = 1/(2 × (| 1/λ851 - 1/λ852|)).

3. Appareil de traitement par énergie selon la revendication 1,
dans lequel, quand la distance entre l'unité d'émission (3) et le réflecteur (6) est L, les longueurs d'onde (λ851, λ852) des énergies rayonnées respectives sont choisies de telle manière qu'un entier fois la moitié de la longueur d'onde de chacune des ondes stationnaires (851, 852) est égal à la distance (L) entre l'unité d'émission (3) et le réflecteur (6), c'est-à-dire L = n × λ851 / 2 et L = m × λ852 / 2, où n et m sont des entiers.

4. Appareil de traitement par énergie selon la revendication 1,
dans lequel, quand les longueurs d'onde (λ851, λ852) des énergies rayonnées respectives sont présentes ou connues, la distance (L) entre l'unité d'émission (3) et le réflecteur (6) est maîtrisée de telle manière que les longueurs d'onde (λ1, λ2) satisfont les formules L = n × λ851 / 2 et L = m × λ852 / 2, où n et m sont des entiers.

5. Appareil de traitement par énergie selon la revendication 1,
dans lequel l'unité d'émission d'énergie (3) comprend plusieurs unités d'émission pour émettre des énergies de différentes fréquences.
